# EUROPEAN PATENT SPECIFICATION

(11) **EP 3 152 231 B1**
(45) Date of publication and mention of the grant of the patent: **03.11.2021**
(21) Application number: 15728124.7
(22) Date of filing: 08.06.2015
(51) Int. Cl.: C07K 14/705, G01N 33/564, A61K 38/00

(54) **PEPTIDES, AND METHODS AND APPARATUS UTILISING SAME**
PEPTIDE SOWIE VERFAHREN UND VORRICHTUNG DAMIT
PEPTIDES, ET PROCÉDÉS ET APPAREIL LES UTILISANT

(30) Priority: 06.06.2014 GB 201410108
(43) Date of publication of application: 12.04.2017
(73) Proprietor: The University Of Manchester, Manchester M13 9PL (GB)
(72) Inventor: BRENCHLEY, Paul, Sale Cheshire M33 5ED (GB); MCKENZIE, Edward, High Peak Derbyshire SK22 1BT (GB); GUMMADOVA, Jennet, Runcorn Cheshire WA7 1XD (GB); FRESQUET, Maryline, Sale M33 3AY (GB)
(74) Representative: HGF
(86) International application number: PCT/GB2015/051662
(87) International publication number: WO 2015/185949

(56) References cited:
- WO-A1-2010/009457
- WO-A1-2013/007640
- WO-A2-2005/066337
- US-A1- 2011 065 647
- DURGA KANIGICHERLA ET AL: "Anti-PLA2R antibodies measured by ELISA predict long-term outcome in a prevalent population of patients with idiopathic membranous nephropathy", KIDNEY INTERNATIONAL, vol. 83, no. 5, 1 May 2013 (2013-05-01), pages 940-948, XP055208680, ISSN: 0085-2538, DOI: 10.1038/ki.2012.486
- M. FRESQUET ET AL: "Identification of a Major Epitope Recognized by PLA2R Autoantibodies in Primary Membranous Nephropathy", JOURNAL OF THE AMERICAN SOCIETY OF NEPHROLOGY., vol. 26, no. 2, 6 October 2014 (2014-10-06), pages 302-313, XP55224195, US ISSN: 1046-6673, DOI: 10.1681/ASN.2014050502
- None

## Description

### Field of the invention

The present invention relates to peptides that are able to bind to anti-PLA2R antibodies, and also to such peptides for use in the prevention or treatment of kidney disease. The invention also relates to pharmaceutical compositions comprising a peptide and a pharmaceutically acceptable carrier. The invention relates to the use of peptides that are able to bind to anti-PLA2R antibodies in methods of preventing or treating kidney disease in a subject by providing a therapeutically effective amount of a peptide to a subject in need of such prevention or treatment, and to devices for extracorporeal treatment of a patient's blood, as well as methods of determining levels of anti-PLA2R antibodies in a subject.

### Background

Idiopathic membranous nephropathy (IMN) is a rare form of glomerulonephritis affecting 10-12 cases per million population. The significant discovery in 2009 that circulating antibodies to phospholipase A2 receptor 1 (PLA2R) are present in 70% of patients with IMN, identified the autoimmune nature of this pathology. Genetic evidence of the involvement of PLA2R in IMN came from the genome wide association study identifying *PLA2R* and *DQA1* as genes accountable for the genetic susceptibility to IMN. Clinical confirmation that anti-PLA2R antibodies are relevant in MN is evident from studies showing an association between high levels of anti-PLA2R and active disease, poor clinical outcome at 5 years and less chance of spontaneous remission.

In other autoimmune kidney diseases such as anti-GBM disease, which is characterized by anti-collagen IV (α3NC1) autoantibodies and ANCA vasculitis characterized by anti-MPO autoantibodies, the antigenic epitopes include both linear peptide sequences and 3D conformational structure. Knowledge of these antigen epitopes in these diseases has been important in understanding the pathological disease mechanisms and may help to classify patient subgroups and severity of disease. However, specific experimental structural data for PLA2R is currently lacking, although this is required to improve the prospects of targeted treatments for patients with IMN.

The interactions between anti-PLA2R and PLA2R are reported to be sensitive to chemical reducing agents, stable in the detergent SDS and independent of glycosylation. However, it is unlikely that there are genetically determined amino acid changes in PLA2R specific to cases of IMN that could influence immunogenicity or contribute to a 3D conformational disease epitope. One study described linear epitopes within PLA2R using an overlapping array of short peptide sequences derived from PLA2R. Seven linear peptides were tested using an ELISA assay but no difference was seen comparing IMN seropositive patients and control sera. This suggests that 3D conformational structure is a critical feature of PLA2R epitopes. Yet, the location and nature of epitopes recognized by anti-PLA2R are unknown.

Peptides comprising large fragments of PLA2R for use in the prognosis, detection, treatment or prevention of kidney disease, including IMN has been published in patent application WO2010009457A1. Additionally, further domains of PLA2R for use in the prognosis, detection or treatment of kidney cancer were published in patent application WO2013007640. A study by Durga Kanigicherla *et al.* (2013), also describes a peptide comprising PLA2R domains for use in the prognosis and detection of kidney disease. It is also known that full length PLA2R protein is difficult to synthesise in quantities that allow its wide spread use in clinical and commercial applications. The inventors have identified a specific epitope having 40 amino acids or less that is capable of binding PLA2R anti-bodies, providing a clear benefit since such peptides may be produced by easier and more cost effective ways than have previously been possible in respect of PLA2R itself. In addition, they are less immunogenic than the full-length protein due to their smaller size.

### Summary of the invention

In a first aspect, the invention provides a peptide consisting of a length of up to 40 amino acid residues, the peptide comprising the amino acid sequence of K-X1-X2-X3-X4-X5-K-X6-X7-X8-X9-X10-X11-X12-X13-K(SEQ ID NO:2) wherein X1 and X13 are both cysteine residues and wherein the amino acid sequence set forth in SEQ ID NO:2 shares at least 80% identity with SEQ ID NO: 3 over the entire length of the sequence.

In an embodiment, the peptide according to the first aspect of the invention has a length of 31 amino acid residues.

In an embodiment, the peptide according to the first aspect:
i) comprises additional amino acid residues located at the N terminal of SEQ ID NO:2;
ii) comprises a fragment of PLA2R comprising the sequence from amino acid residues 50 to 65 of the full-length protein (SEQ ID NO:3);
iii) consists of SEQ ID NO:3;
iv) comprises or consists of a fragment of PLA2R comprising the sequence from amino acid residues 35 to 65 of the full-length protein (SEQ ID NO:4);
v) comprises at least one further amino acid sequence from the group consisting of: a sequence from the Fibronectin II domain of PLA2R; a sequence from a CTLD domain of PLA2R, such as a sequence from the CTLD1 domain of PLA2R, a sequence from the CTLD2 domain of PLA2R, and a sequence from the CTLD3 domain of PLA2R,
   optionally wherein:
   i) the sequence from the Fibronectin II domain of PLA2R comprises the sequence EDDLLWCATTSR; or
   ii) the sequence from the CTLD domain of PLA2R comprises a sequence selected from the group consisting of: YLNHIDHEIVEKDAWK; YYATHCEPGWNPYNR; TWHEALR; AGHVLSDAESGCQEGWER; and YSGGCVAMRGR;
vi) shares at least 80% identity with a SEQ ID NO:4.

In a second aspect, the invention provides a peptide in accordance with the present invention for use in the prevention or treatment of kidney disease.

In a third aspect, the invention provides a pharmaceutical composition comprising a peptide of the present invention and a pharmaceutically acceptable carrier.

In an fourth aspect the invention provides a device for extracorporeal treatment of a patient's blood, the device comprising:
- a peptide in accordance with the present invention; and
- means for separating the peptide from blood.

In an embodiment of a device according to the fourth aspect of the invention wherein:
i) the means for separating the binding partner from blood comprises a substrate of a material selected from the group consisting of: cellulose; cellulose derivatives; agarose; agarose derivatives; polysulphone; polysulphone derivatives; polyacrylamide; polyacrylamide derivatives; and nylon; and/or
ii) the device is provided in a form selected from the group consisting of: hollow fibre cassettes, membranes, and beads.

An embodiment provides a peptide according to the first aspect of the invention for use in the treatment or prevention of kidney disease, wherein the kidney disease is selected from the group consisting of: primary renal failure; membranous nephropathy, such as idiopathic membranous nephropathy or de novo membranous nephropathy; and focal segmental glomerulosclerosis.

In a fifth aspect the invention provides a method of determining levels of anti-PLA2R antibodies in a subject, the method comprising:
- contacting a peptide in accordance with the present invention with a sample of a body fluid from the subject, such that anti-PLA2R antibodies present in the subject's body fluid sample are able to bind to and be retained by the peptide; and
- determining the amount of anti-PLA2R antibodies retained by the peptide.

In a suitable embodiment of a method of the fifth aspect of the invention, the invention provides a method of diagnosing kidney disease in a subject, the method comprising:
- determining *in vitro* the amount of anti-PLA2R antibodies in a body fluid sample from a subject in a method of the fifth aspect of the invention; and
- comparing the determined amount with a reference value,
wherein if the determined amount of anti-PLA2R antibodies in the sample is equal to or greater than the reference value, this indicates that the subject has kidney disease.

In a suitable embodiment of a method of the fifth aspect of the invention, the subject is one that is considered to be at risk of kidney disease.

In a suitable embodiment of a method of the fifth aspect of the invention, the invention provides a method of selecting a suitable regimen for the prevention or treatment of kidney disease, the method comprising:
- determining the amount of anti-PLA2R antibodies in a body fluid sample from a subject in a method of the fifth aspect of the invention; and
- comparing the determined amount with a reference value,
wherein if the determined amount of anti-PLA2R antibodies in the sample is equal to or greater than the reference value, this indicates that the subject will benefit from a regimen for prevention or treatment of kidney disease utilising a binding partner for an anti-PLA2R antibody.

In a suitable embodiment of a method of the fifth aspect of the invention:
i) the subject is an individual diagnosed as having kidney disease, where the cause of the kidney disease has not been determined; and/or
ii) the method further comprises obtaining a value representative of the amount of anti-PLA2R antibodies in the subject's body fluid, and comparing this obtained value with a reference value, wherein if the obtained value is larger than the reference value this indicates that the subject will benefit from a regimen for prevention or treatment of kidney disease utilising a peptide according to the first aspect of the invention.

In a suitable embodiment, a method according to the fifth aspect of the invention for use in monitoring effectiveness in a subject of a treatment regimen for prevention or treatment of kidney disease, the method comprising:
- assaying a sample of a body fluid of a subject undergoing a treatment regimen for prevention or treatment of kidney disease, to determine the amount of anti-PLA2R antibodies present in the subject's body fluid sample by a method according to the fifth aspect of the invention;
- comparing the determined amount with a reference value; and
- based on this comparison, determining whether the subject's treatment regimen is effective for prevention or treatment of kidney disease.

In a suitable embodiment, the reference value is the mean plus three standard deviations of the amount of anti- PLA2R antibody in approximately 30 samples from normal healthy individuals without kidney disease or the reference value is representative of the amount of anti- PLA2R antibodies present in a comparable sample from the subject at an earlier time point;
ii) wherein the subject is a patient undergoing therapy for kidney disease;
iii) wherein the subject is a participant in a clinical trial.

As discussed further below, these methods of the fifth aspect of the invention may have many applications including use in diagnosing kidney disease in a subject, in selecting a suitable regimen for the prevention or treatment of kidney disease, or monitoring effectiveness in a subject of a treatment regimen for the prevention or treatment of kidney disease.

### Detailed description of the invention

Certain disclosures are based upon the surprising finding that peptides comprising the amino acid sequence K-X1-X2-X3-X4-X5-K-X6-X7-X8-X9-X10-X11-X12-X13-K (SEQ ID NO:2) are capable of exerting the major antibody-binding activity of the full-length PLA2R protein. The lysine residues, which have a spacing corresponding to the lysine residues found at positions 50, 56, and 65 of the full-length native human protein, contribute significantly to this binding activity.

It will be appreciated that the ability of peptides of invention to bind to anti-PLA2R antibodies, and particularly to compete with naturally occurring PLA2R for the binding of the autoantibodies that occur in patients with kidney disease, means that these peptides have many useful applications. The binding of anti-PLA2R autoantibodies to PLA2R *in vivo* is a key mechanism contributing to the development of, and damage associated with, kidney diseases such as IMN. As referred to in the second to fifth aspects of the invention mentioned above, and as described in further detail below, peptides of the invention have uses in the prevention or treatment of kidney diseases, and in diagnosis of such diseases. As described herein, a therapeutically effective amount of a peptide according to the invention is an amount of such a peptide that provides a therapeutically effective inhibition of binding of anti-PLA2R antibodies to native PLA2R in the subject.

The full-length PLA2R protein has proved notably difficult to synthesise in quantities that would allow its wide spread use in clinical and commercial applications such as the treatments or diagnostic methods considered above. Furthermore, until the inventors' current finding that the major antibody-binding activity of PLA2R can be exerted by the peptides of the invention, the location of the major relevant epitope in PLA2R has been unknown. This has effectively prevented the ability to utilise shorter, more easily synthesised, peptides to replicate PLA2R's antibody-binding activity. On considering the present disclosure, the skilled person will readily appreciate that the finding that the antibody-binding activity of full-length PLA2R can be achieved using much shorter peptides of the present invention provides a clear benefit, since such peptides may be produced by easier and more cost effective ways than have previously been possible in respect of PLA2R itself.

Furthermore, since the peptides of the invention are much smaller, and hence less immunogenic than PLA2R, their use in systemic therapy is not expected to induce the formation of further damaging anti-PLA2R antibodies, an outcome that may be expected were full-length PLA2R to be used in this manner.

The various aspects and embodiments of the invention will be described further below to aid in the understanding of the invention. In keeping with this aim, certain terms used in the disclosure are further described or defined in the following paragraphs.

### Peptides of the invention

In order to provide the various beneficial activities referred to above, a peptide of the invention must exhibit the ability to bind to anti-PLA2R antibodies. Tests by which such binding may be verified include enzyme linked immunosorbent assays (ELISA), other coated surface assays, such as bead immunoassays or surface plasmon resonance, or the immuno-blotting approach described further in the Examples set out below.

For the purposes of the present disclosure, the peptides of the invention should not be taken as encompassing full-length PLA2R itself. As described further below, peptides of the invention may comprise fragments of PLA2R, or variants of such fragments.

Also disclosed herein, the peptides of the disclosure comprise the residues X1 to X13 that may be independently selected from any naturally, or non-naturally, occurring amino acid residue.

The peptides of the invention comprise at least two cysteine residues,whereinX1 and X13 are both cysteine residues. The inventors have found that the presence of disulphide bridges within the peptides of the invention facilitates the presentation of the lysine residues in a manner that allows their ability to bind to anti-PLA2R antibodies. Thus peptides in accordance with these embodiments of the invention are able to bestow this advantage.

A peptide of the invention has a length of up to 40 amino acid residues. In a suitable embodiment a peptide of the invention has a length of 40 amino acid residues. In a suitable embodiment a peptide of the invention has a length of 39 amino acid residues. In a suitable embodiment a peptide of the invention has a length of 38 amino acid residues. In a suitable embodiment a peptide of the invention has a length of 37 amino acid residues. In a suitable embodiment a peptide of the invention has a length of 36 amino acid residues. In a suitable embodiment a peptide of the invention has a length of 35 amino acid residues. In a suitable embodiment a peptide of the invention has a length of 34 amino acid residues. In a suitable embodiment a peptide of the invention has a length of 33 amino acid residues. In a suitable embodiment a peptide of the invention has a length of 32 amino acid residues. In a suitable embodiment a peptide of the invention has a length of 31 amino acid residues. In a suitable embodiment a peptide of the invention has a length of up to 30 amino acid residues. In a suitable embodiment a peptide of the invention has a length of 30 amino acid residues. In a suitable embodiment a peptide of the invention has a length of 29 amino acid residues. In a suitable embodiment a peptide of the invention has a length of 28 amino acid residues. In a suitable embodiment a peptide of the invention has a length of 27 amino acid residues. In a suitable embodiment a peptide of the invention has a length of 26 amino acid residues. In a suitable embodiment a peptide of the invention has a length of 25 amino acid residues. In a suitable embodiment a peptide of the invention has a length of up to 20 amino acid residues. In a suitable embodiment a peptide of the invention has a length of up to 19 amino acid residues. In a suitable embodiment a peptide of the invention has a length of up to 18 amino acid residues. In a suitable embodiment a peptide of the invention has a length of 17 amino acid residues.

In such peptides of the invention, which comprise more than the 16 residues of SEQ ID NO:2, the additional amino acid residues may be located at the N terminal of SEQ ID NO:2., A first amino acid residue added to the N terminal of SEQ ID NO:2 may be termed X-1, a second amino acid residue added to the N terminal of SEQ ID NO:2 X-2, and a third amino acid residue added to the N terminal of SEQ ID NO:2 X-3, etc.

A peptide of the invention may suitably comprise 20 or less amino acid residues located at the N terminal of SEQ ID NO:2 (i.e. X-20 to X-1 in addition to SEQ ID NO:2). A peptide of the invention may suitably comprise 15 or less amino acid residues located at the N terminal of SEQ ID NO:2 (i.e. X-15 to X-1 in addition to SEQ ID NO:2). A peptide of the invention may suitably consist of SEQ ID NO:2 and 15 amino acid residues located at the N terminal of SEQ ID NO:2. A peptide of the invention may suitably comprise 10 or less amino acid residues located at the N terminal of SEQ ID NO:2 (i.e. X-10 to X-1 in addition to SEQ ID NO:2). A peptide of the invention may suitably comprise 5 or less amino acid residues located at the N terminal of SEQ ID NO:2 (i.e. X-5 to X-1 in addition to SEQ ID NO:2).

The inventors have found that embodiments in which additional amino acids are located at the N terminal of SEQ ID NO:2 are particularly effective in binding anti-PLA2R antibodies.

In a suitable embodiment one or more of amino acid residues X1 to X13 may be independently selected from the following peptides: a peptide of the invention in which X1 is C; a peptide of the invention in which X2 is I; a peptide of the invention in which X3 is Q; a peptide of the invention in which X4 is A; a peptide of the invention in which X5 is G; a peptide of the invention in which X6 is S; a peptide of the invention in which X7 is V; a peptide of the invention in which X8 is L; a peptide of the invention in which X9 is T; a peptide of the invention in which X10 is L; a peptide of the invention in which X11 is E; a peptide of the invention in which X12 is N; a peptide of the invention in which X13 is C.

In a suitable embodiment a peptide of the first aspect of the invention is a fragment of PLA2R comprising the sequence from amino acid residues 50 to 65 of the full-length protein. The amino acid sequence of this fragment is set out in SEQ ID NO:3.

In a suitable embodiment a peptide of the first aspect of the invention is a fragment of PLA2R consisting of the sequence from amino acid residues 50 to 65 of the full-length protein (SEQ ID NO:3).

In a suitable embodiment a peptide of the invention is a fragment of PLA2R comprising the sequence from amino acid residues 35 to 65 of the full-length protein. As shown in the Examples set out elsewhere in this disclosure, this 31 amino acid residue peptide (the amino acid sequence of which is set out in SEQ ID NO:4) shows a great degree of specific binding to anti-PLA2R antibodies. As a consequence, the peptide of SEQ ID NO:4 is able to significantly inhibit the binding of anti-PLA2R autoantibodies to full-length PLA2R. The results reported in the Examples section illustrate that the presence of the peptide of SEQ ID NO:4 is able to inhibit binding of anti-PLA2R antibodies to PLA2R by 83%. As discussed above, the binding of anti-PLA2R autoantibodies to PLA2R *in vivo* contributes to the development of, and damage associated with, kidney diseases such as IMN.

Described herein is a peptide that comprises a fragment of the ricin domain of PLA2R. Indeed, the peptide may consist of a fragment of the ricin domain of PLA2R.

In a suitable embodiment a peptide of the invention may comprise a at least one further amino acid sequence from the group consisting of: a sequence from the Fibronectin II domain of PLA2R; a sequence from a CTLD domain of PLA2R, such as a sequence from the CTLD1 domain of PLA2R, a sequence from the CTLD2 domain of PLA2R, and a sequence from the CTLD3 domain of PLA2R.

In a suitable embodiment, a sequence from the Fibronectin II domain of PLA2R to be included in a peptide of the invention may comprise the sequence EDDLLWCATTSR.

In a suitable embodiment, a sequence from a CTLD domain of PLA2R to be included in a peptide of the invention may comprise a sequence selected from the group consisting of: YLNHIDHEIVEKDAWK; YYATHCEPGWNPYNR; TWHEALR; AGHVLSDAESGCQEGWER; and YSGGCVAMRGR.

Without wishing to be bound by any hypothesis, the inventors believe that presence of such amino acid sequences in the peptides of the disclosure contributes to the adoption of a conformation that presents residues, including the lysine residues that interact with anti-PLA2R antibodies, in a manner that promotes antibody binding.

In a suitable embodiment a peptide of the invention is a fragment of PLA2R consisting of the sequence from amino acid residues 35 to 65 of the full-length protein (SEQ ID NO:4). The peptide of SEQ ID NO:4 consists of amino acid residues found in the ricin domain of PLA2R.

In a suitable embodiment a peptide of the invention is an anti-PLA2R antibody-binding fragment of SEQ ID NO:4. Techniques for use in the assessment of antibody binding are discussed elsewhere in the specification.

It will be appreciated that fragments of PLA2R other than those set out above may also constitute peptides of the invention, as long as these meet the definition set out in the first aspect of the invention.

As an alternative to a fragment of PLA2R, a peptide of the disclosure may comprise a variant of a fragment of PLA2R. For the present purposes, a "variant" should be taken as a peptide that meets the definition SEQ ID NO:2, and shares at least 50% identity with a corresponding fragment of PLA2R. Merely by way of example, a peptide that comprises SEQ ID NO:2, and in which five of X1 to X13 correspond to the amino acid residues found at positions 51 to 55, and 57 to 64, of full-length PLA2R will constitute a variant of the fragment spanning residues 50 to 65 of full-length PLA2R (since, taking into account correspondence between the lysine residues required by SEQ ID NO:2, and those found at positions 50, 56, and 65 of full-length PLA2R, the degree of identity between such a peptide and the corresponding PLA2R sequence will be 50%).

Such peptides representing variants of the native PLA2R sequence are not limited to variants of the sequence spanning residues 50 to 65 of PLA2R. A suitable peptide of the disclosure may, for example, comprise or consist of a variant sharing at least 50% identity with SEQ ID NO:4.

Variants of the sort set out above, may share a greater degree of identity with the sequence of native PLA2R. In a suitable embodiment a peptide of the invention shares at least 80% identity with a corresponding fragment of PLA2R such as SEQ ID NO:3 or SEQ ID NO:4. In a suitable embodiment a peptide of the invention shares at least 85% identity with a corresponding fragment of PLA2R such as SEQ ID NO:3 or SEQ ID NO:4. In a suitable embodiment a peptide of the invention shares at least 90% identity with a corresponding fragment of PLA2R such as SEQ ID NO:3 or SEQ ID NO:4. In a suitable embodiment a peptide of the invention shares at least 95% identity with a corresponding fragment of PLA2R such as SEQ ID NO:3 or SEQ ID NO:4. In a suitable embodiment a peptide of the invention may share up to 99%, or more, identity with a corresponding fragment of PLA2R such as SEQ ID NO:3 or SEQ ID NO:4.

The amino acid sequence of a peptide of the invention may differ from the amino acid sequence of a corresponding fragment of PLA2R by one or more amino acid residues, by two or more amino acid residues, by three or more amino acid residues, by four or more amino acid residues, or by five or more amino acid residues. The amino acid sequence of a peptide of the invention may differ from the amino acid sequence of a corresponding fragment of PLA2R by ten or more amino acid residues, by fifteen or more amino acid residues, by 20 or more amino acid residues, or by 25 or more amino acid residues, so long as the requirements of SEQ ID NO:2 are met.

Variants of SEQ ID NO:4 constitute particularly useful peptides of the invention. Merely by way of example, such variants may preferably correspond to the sequence of SEQ ID NO:4 save for substitution of one, two, three, four, or five amino acid residues (other than the lysine residues at the positions corresponding to residues 50, 56, or 65 of full-length PLA2R).

Disclosed herein is a peptide comprising the amino acid sequence GIFVIQSESLKK (also referred to herein as "peptide 2" or SEQ ID NO:5).

### Kidney disease

Specific examples of "kidney disease" that may be diagnosed, prevented or treated by the methods, uses or medicaments of the invention may be selected from the group consisting of: primary renal failure; membranous nephropathy, such as idiopathic membranous nephropathy, or *de novo* membranous nephropathy; and focal segmental glomerulosclerosis.

The binding of anti-PLA2R autoantibodies to PLA2R *in vivo* is a key mechanism contributing to the development of, and damage associated with, kidney diseases. The inventors believe that the various aspects and embodiments of the invention described herein are able to beneficially reduce such binding, thereby providing a desirable therapeutic effect.

### Body fluid samples

Certain embodiments of the aspects of the invention utilise samples of a body fluid from a subject to determine the amount of anti-PLA2R antibodies present therein.

In a suitable embodiment, the body fluid is selected from the group consisting of: blood; serum; plasma; urine; saliva; and interstitial fluid.

The sample may be one that is obtained from the subject, for example by provision of a blood or interstitial fluid sample, or provision of a urine sample, and this step of obtaining the sample may optionally constitute part of the method in question.

### A subject

For the purposes of the present invention, a subject may be an individual identified as having, or potentially as having (for example, as at risk of having) kidney disease. The nature of an appropriate subject will be apparent to a skilled reader depending on the aspect of the invention in question.

Merely by way of example, in the case of a method for use in diagnosing kidney disease in accordance with the invention, a suitable subject may be one that is considered to be at risk of kidney disease. This assessment of risk may be based upon pre-disposing factors, such as genetic predisposition to kidney disease, or on the exhibition of symptoms consistent with kidney disease. Alternatively, a suitable subject for a method for use in diagnosing kidney disease in accordance with the invention may be an individual exhibiting clinical symptoms of kidney disease, but where the cause or nature of the kidney disease has not been identified.

For illustration purposes, in a method of preventing or treating kidney disease as described herein, a suitable subject may be one diagnosed as having kidney disease (for example by a method of the invention) or as exhibiting symptoms consistent with kidney disease. Indeed, the subject may be an individual where this regimen for prevention or treatment has been selected as being suitable by a method of the invention.

In the case of the invention's methods for use in selecting a suitable regimen for the prevention or treatment of kidney disease, a suitable subject may be an individual diagnosed as having kidney disease, but where the cause of the kidney disease has not been determined. In the event that the method of the invention indicates that anti-PLA2R antibodies are present within the subject, this suggests that these antibodies are contributing to the disease process, and that a regimen utilising a binding partner for an anti-PLA2R antibody in a therapeutic manner is likely to be suitable for prevention or treatment of the kidney disease.

In the case of a method for use in monitoring effectiveness in a subject of a treatment regimen for prevention or treatment of kidney disease, a suitable subject may be a patient undergoing therapy for kidney disease (for example by a method of prevention or treatment of the disclosure, or by pharmaceutical compositions or medical uses of the invention). In this case, the method of the invention may be used as part of a process of ongoing care for the subject, with a view to optimising a regimen for use in prevention or treatment of the disease, or ensuring that a selected regimen remains effective. Alternatively, it will be recognised that methods for use in monitoring effectiveness in a subject are also suitable for use in the development of novel prevention or treatment regimens, such as by clinical trials.

Also disclosed herein are methods of preventing or treating kidney disease practiced in respect of subjects requiring such prevention or treatment. A suitable subject requiring such prevention or treatment may be one diagnosed as having kidney disease, or otherwise exhibiting symptoms consistent with the presence of kidney disease.

### Specific embodiments of methods of the fifth aspect of the invention

The fifth aspect the invention provides methods of determining levels of anti-PLA2R antibodies in a subject. These methods may be put to various beneficial uses, including, but not limited to, the embodiments set out below.

Methods of the fifth aspect of the invention utilise a peptide of the invention as a binding partner for anti-PLA2R antibodies in a body fluid sample from a subject. In a suitable embodiment a method of this aspect of the invention may additionally employ one or more further binding partners that bind to and retain anti-PLA2R antibodies. Suitably such further binding partners may include full-length PLA2R, or fragments of PLA2R such as the N-C3 fragment described further in the examples.

In a suitable embodiment of a method of the fifth aspect of the invention, the invention provides a method of diagnosing kidney disease in a subject, the method comprising:
- determining the amount of anti-PLA2R antibodies in a body fluid sample from a subject in a method of the fifth aspect of the invention; and
- comparing the determined amount with a reference value,
wherein if the determined amount of anti-PLA2R antibodies in the sample is equal to or greater than the reference value, this indicates that the subject has kidney disease.

In a suitable embodiment of a method of the fifth aspect of the invention, the invention provides a method of selecting a suitable regimen for the prevention or treatment of kidney disease, the method comprising:
- determining the amount of anti-PLA2R antibodies in a body fluid sample from a subject in a method of the fifth aspect of the invention; and
- comparing the determined amount with a reference value,
wherein if the determined amount of anti-PLA2R antibodies in the sample is equal to or greater than the reference value, this indicates that the subject will benefit from a regimen for prevention or treatment of kidney disease utilising a binding partner for an anti-PLA2R antibody.

In a suitable embodiment, the binding partner for anti-PLA2R antibodies is a peptide in accordance with the present invention.

In a suitable embodiment of a method of the fifth aspect of the invention, the invention provides a method of monitoring effectiveness in a subject of a treatment regimen for prevention or treatment of kidney disease, the method comprising:
- assaying a sample of a body fluid of a subject undergoing a treatment regimen for prevention or treatment of kidney disease, to determine the amount of anti-PLA2R antibodies present in the subject's body fluid sample by a method of the fifth aspect of the invention;
- comparing the determined amount with a reference value; and
- based on this comparison, determining whether the subject's treatment regimen is effective for prevention or treatment of kidney disease.

As discussed further herein, methods in accordance with the fifth aspect of the invention may optionally further comprise a step of implementing a suitable regimen for the prevention or treatment of kidney disease. More details of these embodiments are provided elsewhere in the specification.

Alternatively or additionally, the methods of the disclosure may further comprise assessment of one or more additional markers indicative of kidney disease. Examples of such additional markers are considered below.

### Diagnosis of kidney disease

Embodiments of the fifth aspect of the invention provide a method for use in diagnosing kidney disease. The methods in accordance with this aspect of the invention are carried out *in vitro.*

As referred to above, methods for use in diagnosing kidney disease in accordance with the present invention may be useful for the clinical assessment of individuals considered at risk of kidney disease, individuals suffering a disorder that may be kidney disease, or subjects who have already been identified as having kidney disease, but without the underlying cause of the kidney disease having been determined. In these later cases the diagnostic methods of the invention may be useful in determining that the kidney disease is one associated with the presence of anti-PLA2R antibodies.

A method for use in diagnosing kidney disease in accordance with the invention involves determining the amount of anti-PLA2R antibodies present in the subject's body fluid sample, and comparing this obtained value with a reference value. It may then be determined whether or not anti-PLA2R antibodies are present in the sample at a greater amount than in the reference value. In such cases, an obtained value greater than the reference value may be indicative that the subject has kidney disease. Details of suitable reference values that may be employed in such embodiments are discussed elsewhere in the present disclosure.

### Selection of a suitable regimen for the prevention or treatment of kidney disease

One embodiment of the invention comprises a method for the selection of a suitable regimen for the prevention or treatment of kidney disease. The skilled person will appreciate that there are many different forms and causes of kidney disease, as well as many different agents and regimens for the treatment of such disease. Not all treatment regimens are suitable for prevention or alleviation of all forms of kidney disease.

The aspects of the present invention relating to the selection of a suitable treatment or prevention regimen are based upon the inventor's finding that peptides of the invention can be used to bind to anti-PLA2R antibodies generated by a patient's body against the naturally occurring target protein, and allow the levels of these antibodies in a sample to be determined.

By determining whether anti-PLA2R antibodies are present in a subject these methods of the invention make it possible to identify whether or not the subject in question is one who will benefit from treatment aimed at neutralising or removing these antibodies (and thereby preventing further damage that the antibodies may cause) by use of binding partners to the anti-PLA2R antibodies.

Various optional modifications of the methods of the invention are considered within the present disclosure.

### Monitoring effectiveness of a treatment regimen

A suitable embodiment of the fifth aspect of the invention provides a method of monitoring effectiveness in a subject of a treatment regimen for prevention or treatment of kidney disease. As set out elsewhere, embodiments of the methods of the fifth aspect of the invention involve determining the amount of anti-PLA2R antibodies in a sample from a subject, and comparing this with a reference value. Generally, if the determined value is lower than the reference value this indicates that the treatment regimen is effective. In such cases the lower the determined value, as compared to the reference value, the more effective the treatment regimen.

Methods of the invention in accordance with this aspect are suitable for use in a number of different contexts in which it is wished to assess whether or not a treatment regimen is able to effectively prevent or treat kidney disease.

Merely by way of example, methods in accordance with this fifth aspect of the invention are suitable for use in the field of personalised medicine. Here such methods of the invention are able to determine on a patient by patient basis whether or not a proposed treatment regimen proves effective. In the event that a proposed treatment regimen is identified as ineffective it can be modified in an attempt to improve effectiveness, or replaced, and a different regimen tested for efficacy.

These methods of the invention also allow effectiveness of different methods to be determined and compared with one another, wherein the treatment regimen giving rise to the lowest antibody levels will generally be considered the most effective. Thus, even in the event that a given treatment regimen is determined to be effective by the monitoring methods of the present invention, other treatment regimens that are more effective may be identified, and a decision made to adopt a more effective treatment regimen for future prevention or treatment.

As referred to elsewhere, it will also be recognised that methods for use in monitoring effectiveness of a treatment regimen in accordance with this aspect of the invention are also suitable for use in the development of novel prevention or treatment regimens. Here a putative treatment regimen may be adopted, and its effectiveness in reducing anti-PLA2R antibody levels assessed, with those that bring about a reduction selected for future use, or for further refinement. Such methods may be useful in the context of clinical trials or the like.

### Assaying for anti-PLA2R antibodies

The methods of the fifth aspect of the invention involve determining levels of anti-PLA2R antibodies in a subject by determining the amount of anti-PLA2R antibodies retained by a peptide of the invention.

Once informed by the present disclosure that the peptides of the invention are able to bind and retain anti-PLA2R antibodies, the skilled person will be able to determine a number of suitable methods by which assays to determine the amounts of antibody present may be practiced. The following provides some examples of such assays, as well as guidance regarding factors that may be considered in determining a suitable assay to be used in practicing the invention.

Generally, assays for anti-PLA2R antibodies suitable for use in the methods of the invention will be quantitative in nature.

For reference, suitable assays by which the presence of anti-PLA2R antibodies may be detected, and optionally quantitated, include those selected from the group consisting of: enzyme linked immunosorbent assays (ELISAs); western blotting; fluorescent bead-based immunoassays; and immunofluorescence.

Merely by way of example, the ELISA may use an immobilised peptide of the invention, as a binding partner for an anti-PLA2R antibody, to capture anti-PLA2R antibodies in a sample. The captured anti-PLA2R antibodies may then be detected by means of a labelled antibody directed to the immunoglobulin class to which the anti-PLA2R antibodies belong. For example, in the case of anti-PLA2R antibodies comprising IgG, the presence of such antibodies may be determined by use of a labelled (for example peroxidase labelled) anti-human-IgG antibody. Further details of an assay of this sort are set out in the Examples section of this specification.

Assaying of the body fluid sample for anti- PLA2R antibodies is conducted *in vitro.*

### Reference value

The methods for use in monitoring effectiveness in a subject of a treatment regimen for prevention or treatment of kidney disease disclosed herein involve obtaining a value representative of the amount of anti-PLA2R antibodies present in a body fluid sample from a subject, and comparing this obtained value to a reference value.

Furthermore, certain embodiments of methods of the invention optionally involve obtaining a value representative of the amount of anti-PLA2R antibodies present in a body fluid sample, and comparing this value to a reference value.

A number of different values may be used as the reference value in such methods of the invention, and selection of an appropriate reference value will determine the nature of the conclusion that may be drawn using the method of the invention.

In certain embodiments of the invention, a suitable reference value may be one representative of a "background" amount of anti-PLA2R antibodies that may be detectable in a sample without indicating the presence of kidney disease. This baseline may reflect the limit of detection of the assay, or the incidence of false positive results. Reference values of this sort may be suitable for use in the methods disclosed herein.

A suitable reference value reflecting a background level of anti-PLA2R antibodies may be established by assaying approximately 30 samples (for example, 25 or more, 26 or more, 27 or more, 28 or more, 29 or more, or 30 or more samples), from normal healthy individuals without kidney disease, to obtain values representative of the amounts of anti-PLA2R antibodies in these samples. In such an embodiment, the reference value (functioning as a threshold between samples considered positive or negative for kidney disease) may be set at the mean value from the healthy individuals, plus three standard deviations (SDs). In such cases an obtained value above the reference value will be indicative of kidney disease in the subject from whom the value has been obtained, while an obtained value below the reference value will indicate that kidney disease is not present in the subject.

The normal healthy individuals may be matched controls with reference to the subject in respect of whom the method of the invention is to be practiced (for example, the healthy individuals may be matched with respect to gender and/or age), or the healthy individuals may be a panel of individuals (for example, a panel of male and/or female individuals having a range of ages that approximately matches the group in whom kidney disease is to be investigated).

In a suitable embodiment of the methods of the invention, the reference value is representative of the amount of anti-PLA2R antibodies present in a comparable sample from the subject at an earlier time point.

In methods of the fifth aspect of the invention, comparison of the value obtained from the subject with the reference value will provide valuable information as to the effectiveness or ineffectiveness of a treatment regimen. Generally, if the value obtained in respect of the amount of anti-PLA2R antibodies present in the subject's body fluid is lower than the reference value, then this will indicate that the treatment regimen that the subject is undergoing is effective. If the obtained value is higher than the reference value, indicating that the amount of anti-PLA2R antibodies present in the subject's body fluid is elevated as compared to the reference value, this will indicate that the treatment regimen that the subject is undergoing is ineffective. In such cases the treatment regimen may require revision, and the methods of the invention may then be performed again (once the revision has had time to alter the amount of anti-PLA2R antibodies present in the subject) to assess the effectiveness of the revised regimen.

In another example, a first sample is assayed to obtain a first value representative of the amount of anti-PLA2R antibodies present in the subject's body fluid, and a second sample is assayed to obtain a second value representative of the amount of anti-PLA2R antibodies present in the subject's body fluid at a second, later, timepoint, after treatment with a treatment regimen of interest. The first and second obtained values are compared with a reference value indicative of the background level of anti-PLA2R antibodies. If the second obtained value is closer to the reference value than is the first obtained value, then this indicates that the treatment regimen is effective. If the first obtained value is closer to the reference value, or if the two obtained values are the same, this indicates that the treatment regimen is ineffective.

Suitable samples used in the generation of reference values may be of any body fluid, such as sera, as considered elsewhere in the present specification. Samples used for the generation of reference values may by matched, with reference to type, with the sample from which the obtained value is derived. Samples found to contain anti-PLA2R antibodies may be used to create a standard dilution curve as a reference point for comparison with other samples.

### Optional modifications of methods of the invention

The methods of the invention may optionally further comprise selecting a suitable treatment regimen in the event that the subject is diagnosed as having kidney disease.

The methods of the invention may optionally further comprise implementing a suitable treatment regimen in the event that the subject is diagnosed as having kidney disease. The implementation may comprise prescribing the selected treatment regimen. Alternatively, the implementation may comprise providing the selected treatment regimen to the subject.

A suitable treatment regimen in the context of the preceding paragraphs may employ the medical use of binding partners for an anti-PLA2R antibody in accordance with the invention, a method of preventing or treating kidney disease in accordance with the invention, or the use of a device for extracorporeal treatment of a patient's blood in accordance with the invention.

Similarly methods in accordance with the invention may optionally further comprise implementing a suitable prevention or treatment regimen that has been selected by the method of the invention. As above, the implementation may comprise prescribing the selected treatment regimen or providing the selected treatment regimen to the subject. A suitable treatment regimen may employ the medical use of binding partners for an anti-PLA2R antibody in accordance with the invention, a method of preventing or treating kidney disease in accordance with the invention, or the use of a device for extracorporeal treatment of a patient's blood in accordance with the invention.

The methods disclosed herein, may further comprise assessment of one or more additional markers indicative of kidney disease. Such additional markers may be conventional in the art, or may be new markers. Methods utilising combinations of markers may, for example, increase the number of different forms of kidney disease that may be diagnosed by the methods disclosed. Merely by way of example, such additional markers may include antibodies that react with podocyte proteins. Suitably, such additional markers may include one or more markers selected from the group consisting of: anti-IQCJ antibodies; anti-nephrin antibodies; and anti-podocin antibodies.

### Prevention or treatment of kidney disease

The present disclosure, addresses the prevention or treatment of kidney disease in a number of contexts: including selection or monitoring of treatment regimens for the prevention or treatment of kidney disease; methods for use in preventing or treating kidney disease; and medical uses for the prevention or treatment of kidney disease.

Except for where the context requires otherwise, a reference to "treatment" of kidney disease in the present specification should be taken as directed to any effective intervention which has as its purpose the aim of alleviating a disorder manifesting itself as a clinically discernible disease. Treatment, in this context, may encompass any intervention which leads to symptoms of a kidney disease being reduced, or any intervention that prevents the symptoms of kidney disease from worsening in the manner that may be expected if no treatment is undertaken.

For purposes of understanding the present disclosure, references to "prevention" of kidney disease should be taken as directed to intervention initiated before clinical symptoms manifest themselves. Thus methods of preventing kidney disease will be those that avoid the development of clinical symptoms. It will be appreciated that the diagnostic methods of the invention may be of particular benefit in allowing detection of kidney disease before any clinical symptoms are manifest, thereby permitting the use of methods of the invention in order to prevent kidney disease developing.

The prevention or treatment of kidney disease in may be practiced using binding partners for anti-PLA2R antibodies, and such binding partners are discussed further below.

For illustrative purposes, methods of prevention or treatment, medical uses, or devices of the invention may make use of binding partners for anti-PLA2R antibodies (such as peptides according to the invention) as the sole therapeutic agent, or as part of a combination therapy. Suitably such combinations may include immunosuppressive drugs as additional therapeutic agents. Examples of such additional therapeutic agents may be selected from the group consisting of: steroids; cyclophosphamide; cyclosporine; and anti B cell monoclonal antibodies. The binding partners for anti-PLA2R antibodies may be provided preceding, at the same time as, or following treatment with additional therapeutic agents such as immunosuppressive drugs.

### Binding partners for anti-PLA2R antibodies

Binding partners for anti-PLA2R antibodies encompass any agents capable of binding to such antibodies. In the context of the prevention or treatment of kidney disease, the role of such binding partners is to bind to, and thus reduce the adverse impact of, anti-PLA2R antibodies. Any suitable binding partner capable of achieving this activity may be used. Suitably, binding partners may neutralise anti-PLA2R antibodies (for example by binding to the antibody in a manner that prevents the antibody binding to further PLA2R in the subject), or may reduce the amount of the anti-PLA2R antibodies present in the subject.

Suitably, the binding partners utilised will have a preferential affinity for anti-PLA2R antibodies as opposed to other antibodies that may be present. Indeed, the binding partners may be specific for anti-PLA2R antibodies, which is to say that they exhibit minimal, or preferably substantively no, binding of antibodies other than anti-PLA2R antibodies.

The peptides of the present invention are useful as binding partners for anti-PLA2R antibodies. As set out elsewhere in the specification, the peptides of the invention are able to replicate the major antibody-binding activity of PLA2R. However, the use of the peptides of the invention instead of full-length PLA2R in circumstance where it is wished to bind anti-PLA2R antibodies provides a number of significant advantages. Amongst these are that the peptides of the invention are more readily and cost effectively manufactured than full-length PLA2R, and they are also less immunogenic than the full-length protein due to their smaller size.

### Medical uses of the peptides of the invention

The invention provides peptides in accordance with the present invention for use in the prevention or treatment of kidney disease as one of its' aspects. A peptide of the invention for use in accordance with this aspect of the invention may be provided to a subject requiring such prevention or treatment in a therapeutically effective amount.

The skilled person will be able to determine a therapeutically effective amount of peptide of the invention by a range of measures conventional to this field of the art. Merely by way of example, test amounts of a binding partner for an anti-PLA2R antibody may be provided to the subject, and the effectiveness of these various treatment regimens established by a method in accordance with an aspect of the invention.

A peptide of the disclosure for medical use may be provided in a "free" form, in which the peptide is able to move freely within the circulation, bind to anti-PLA2R antibodies, before the peptide-antibody complex is cleared by the body.

A peptide of the disclosure, for medical use in accordance with the disclosure (for example in the methods for prevention or treatment of kidney disease in accordance with the invention) may be provided in a form that is adapted to allow the binding partner, and anti-PLA2R antibodies associated with the binding partner, to be retained.

Also described herein, a binding partner adapted in this manner may be associated with a retention moiety, such as a magnetic bead or the like.

Alternatively, the binding partner may be adapted by immobilisation on a substrate. Suitably, such a substrate may be part of an immunosorbent column. Further details of such embodiments are described elsewhere in this disclosure.

### Methods of prevention or treatment of kidney disease

The present disclosure describes methods of preventing or treating kidney disease in a subject that involve the production of an antibody-depleted volume of blood. The depletion of antibodies from the blood removes agents that cause kidney damage, and the resultant lack of damaging antibodies in the subject's circulation enables the prevention or treatment of kidney disease.

The volume of antibody-depleted blood is, in due course, provided to the subject of the treatment. The blood may be returned immediately to the subject, as considered further below, or may be stored prior to administration to the subject.

There are many different ways in which the step of contacting the volume of the subject's blood with a peptide described herein may be practiced, and suitable examples of these will be readily apparent to those skilled in the art. For instance, the contacting step may be practiced extracorporeally, suitably using a device for extracorporeal treatment of a patient's blood also disclosed herein.

By way of example, a batch of blood comprising one or more volumes of blood to be treated is removed from the subject, and the steps of contacting a volume of the blood with the peptide of the disclosure, and subsequent separation of the peptide of the disclosure and bound antibodies, completed to yield a batch comprising the volume, or volumes, of antibody-depleted blood. Some, or all, of the batch of blood may then be returned to the patient. Alternatively, some or all, of the batch of blood may be stored before return to the patient.

For illustrative purposes, the steps of contacting the blood with the peptide of the disclosure, and subsequent separation, are carried out "in line". Suitably the peptide disclosed is provided in an arrangement so that the patient's blood may flow over the peptide of the disclosure, thus allowing it to bind anti-PLA2R antibodies in the blood. Continued flow of blood then causes separation of the blood from the retained antibody-peptide of the disclosure complex, to yield an antibody-depleted volume of blood. The antibody-depleted volume of blood may then be returned directly to the patient, or retained for future use, as above.

It will generally be that case that the subject from whom the blood has been taken will be the subject requiring the prevention or treatment of kidney disease.

### Devices for extracorporeal treatment of a patient's blood

Devices of the fourth aspect of the invention incorporate means for separating a peptide of the invention from blood, and such devices represent a suitable embodiment that may be employed in the methods of treatment of the invention.

Suitable means for separating the peptide of the invention from the blood may comprise immobilising means. Such immobilising means may comprise a substrate to which the peptide of the invention is attached. In such embodiments passage of blood over the substrate to which the peptide of the invention is attached will allow antibodies present in the blood to attach to the peptide of the invention, and the substrate will retain the peptide of the invention and bound antibody in place. Relative movement of the blood and immobilised peptide of the invention serves to separate antibodies bound to the peptide of the invention from the blood.

For reference purposes, the immobilising means may comprise a retention moiety, such as a magnetic bead or the like, coupled to the peptide of the invention. In this case, the retention moiety may allow immobilisation of the peptide of the disclosure, and thus separation of the bound antibodies from the blood.

Suitable examples of substrates to which a peptide of the disclosure may be attached, and thus immobilised, in devices of the disclosure include those used in immunosorbent columns. In an embodiment, suitable substrates to which a peptide of the invention may be attached include those selected from the group consisting of: cellulose; cellulose derivatives; agarose; agarose derivatives; polysulphone; polysulphone derivatives; polyacrylamide; polyacrylamide derivatives; and nylon. Such substrates may optionally be provided in forms including hollow fibre cassettes, membranes, or beads. In view of the above, it will be appreciated that immunosorbent columns comprising suitable substrates, such as those constituents or forms listed above, represent preferred embodiments of the devices of the fourth aspect of the invention.

The methods, medical uses, and devices disclosed herein are suitable for use in a range of subjects. Kidney disease afflicts not only human subjects, but also animals including domestic cats and dogs. Except for where the context requires otherwise, a suitable subject may generally be selected from the group consisting of: a human subject, a feline subject, and a canine subject.

The invention will now be further described, with reference to the accompanying examples and drawings, in which:

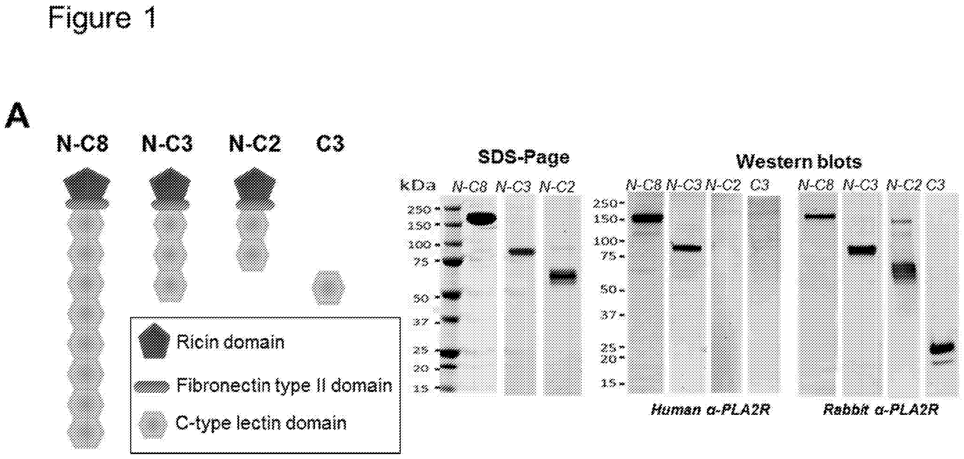

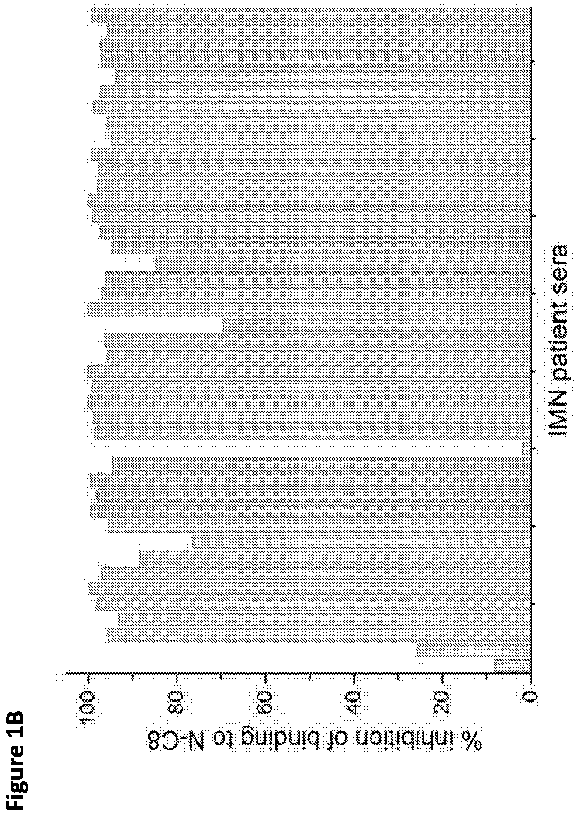

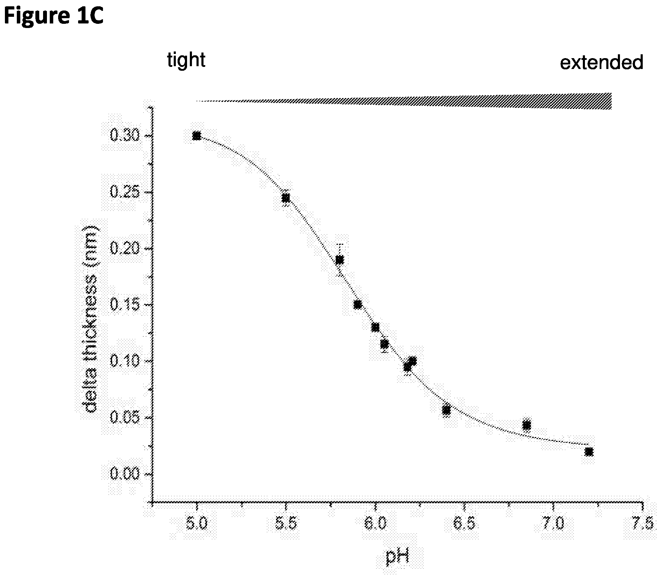

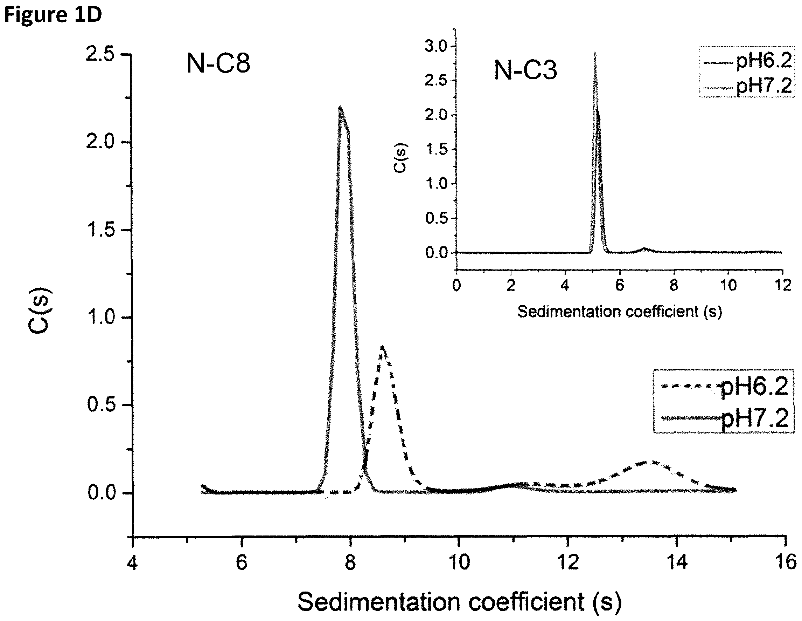

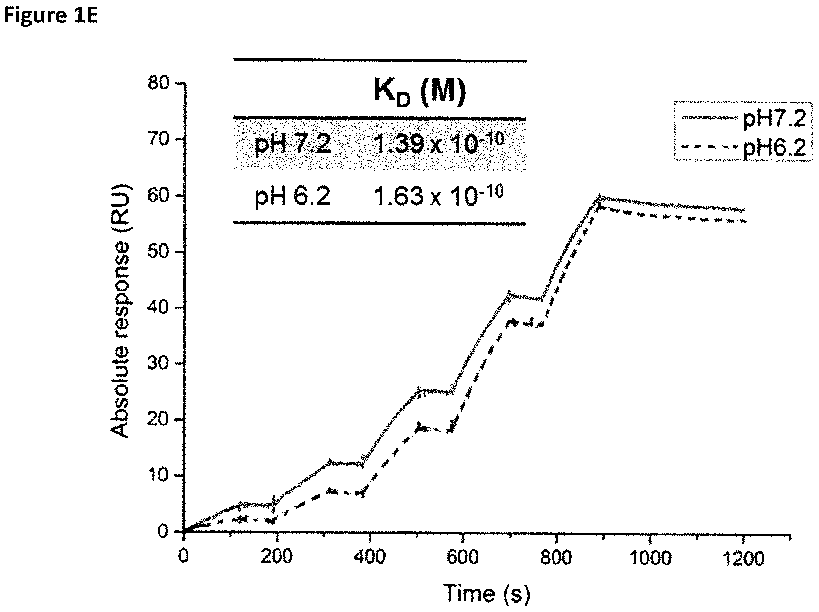

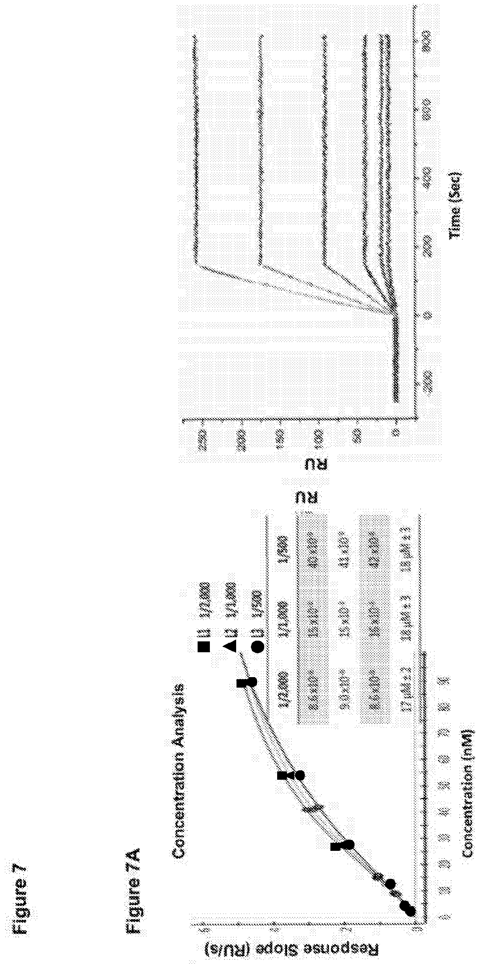

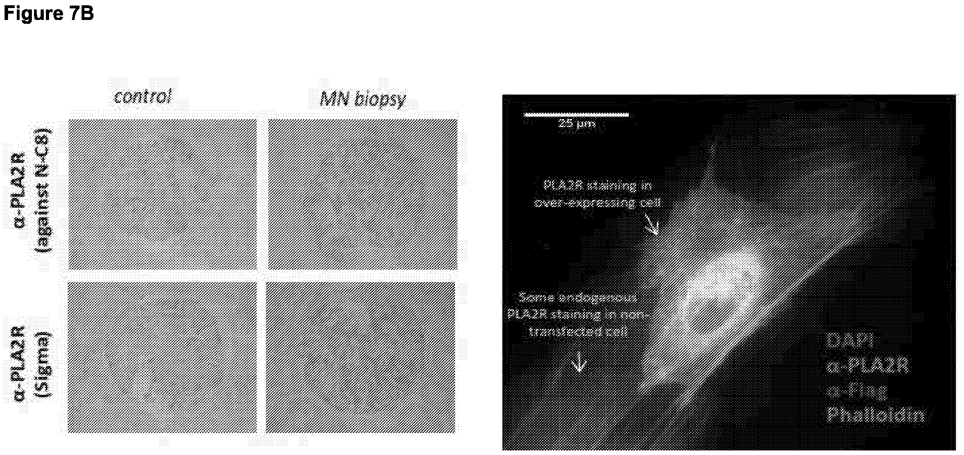

**Figure** 1 - Identification of the major epitope in PLA2R, the effect of pH on its conformation and epitope availability. (A) Representations of the extracellular sub-domains of a long fragment PLA2R (N-C8) comprising the N terminal ricin domain (or cysteine rich domain), fibronectin type II domain and 8 C-type lectin domains (CTLD), two shorter fragments ending after the first 3 C-type lectin domains (N-C3) and the first 2 CTLD (N-C2) and the third CTLD (C3). Silver stained SDS-PAGE gel of purified recombinant N-C8, N-C3 and N-C2 proteins under non-reducing conditions; Lane 1 is a molecular weight marker and subsequent lanes show purified recombinant N-C8 (180 kDa), N-C3 (90 kDa) and N-C2 (65 kDa) proteins. Western blotting analysis of N-C8, N-C3, N-C2 and C3 using human and rabbit antibodies to PLA2R (Figure 7). (B) Competitive ELISA screen of 43 PLA2R-positive patient sera on captured N-C8 inhibited by N-C3. (C) pH dependent conformational change of N-C8 determined by Dual Polarisation Interferometry. Changes in the thickness of the N-C8 layer as a function of decreasing pH was measured and represented the adsorption behaviour of different folded states. (D) Sedimentation velocity of N-C8 and N-C3 (inset) at pH 6.2 (grey line) and 7.2 (black line) analyzed by the distribution of Lamn equation solutions c(s) model using the program Sedfit. (E) Single-cycle kinetics between immobilized purified human PLA2R-specific antibody and N-C8 at pH 6.2 and 7.2. A range of 1, 2, 5, 10 and 15 nM of N-C8 was injected, one cycle at pH 6.2 and the other at pH 7.2. The surface was not regenerated between injections in each cycle.

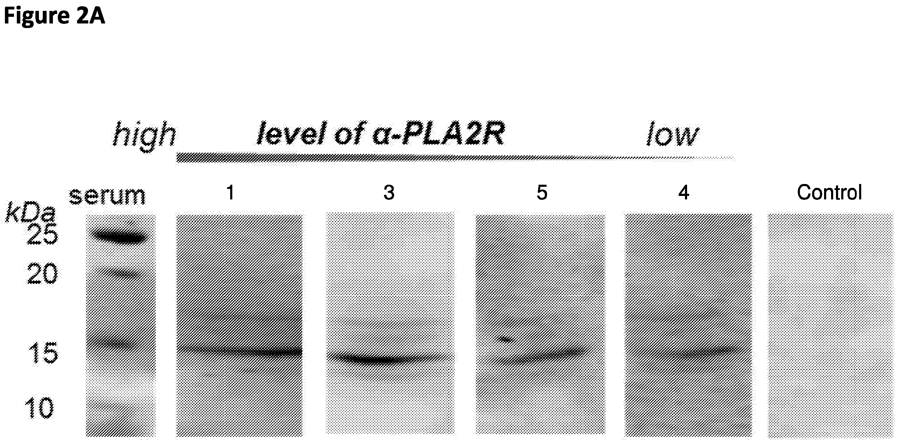

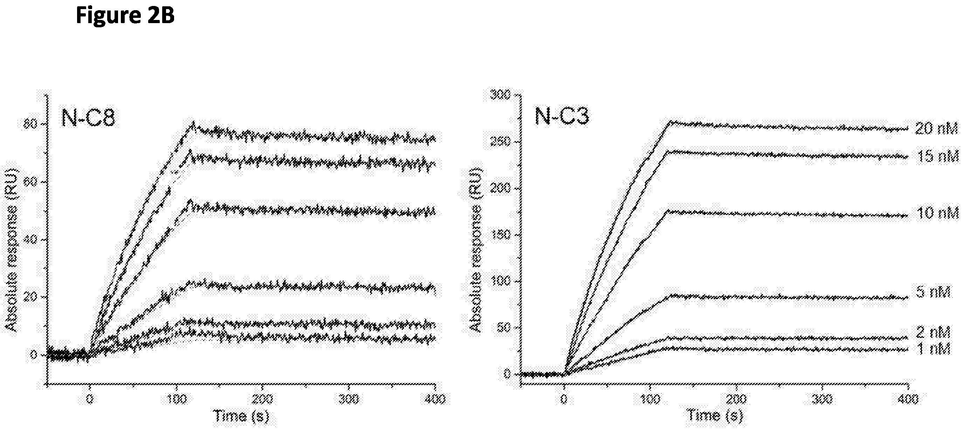

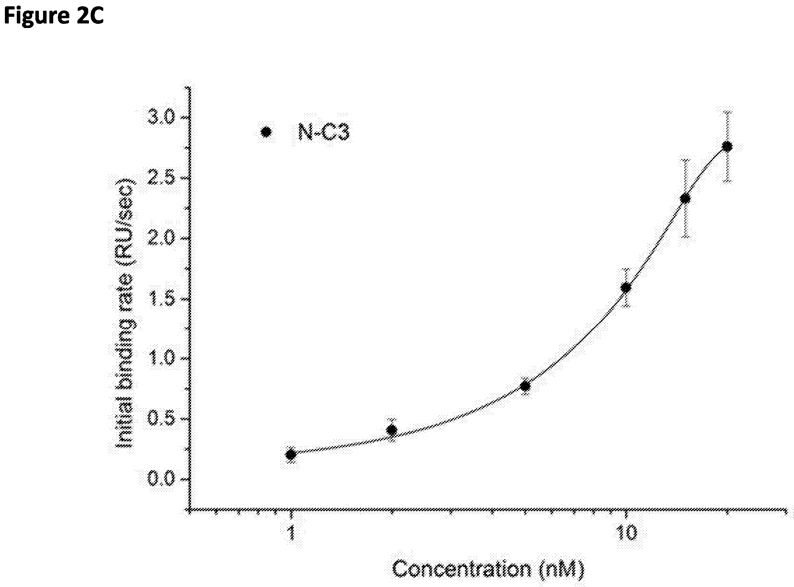

**Figure** 2 - The binding affinity of autoantibodies to N-C8 and N-C3 proteins. (A) Western blot analysis of fragmented N-C8 peptides by OFFGEL fractionation using different patient sera. (B) Representative sensorgrams derived from injections of different concentrations of affinity purified anti-PLA2R antibodies over immobilized N-C8 (on the lefthand panel) and N-C3 (on the right-hand panel). Kinetics data were fitted to a Langmuir 1:1 interaction model (Table 2). (C) Serum anti-PLA2R concentration analysis. Initial association rates of anti-PLA2R binding to immobilized N-C3 plotted as a standard curve used for calculation of the autoantibody concentrations in patient sera (Table 3).

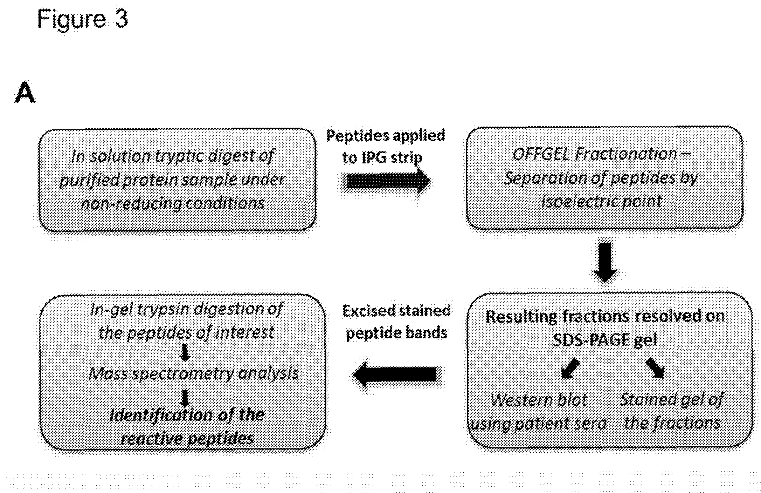

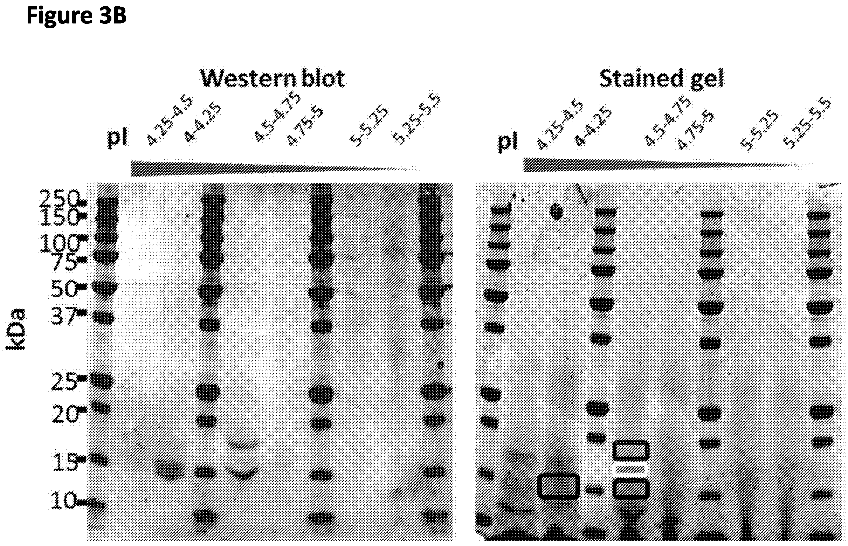

**Figure** 3 - Flow diagram showing experimental details and data analysis. (A) & (B) Purified N-C8 was trypsin digested under non-reducing conditions. The resulting peptides were separated out by charge (OFFGEL fractionation) and molecular weight. The fractions were resolved on a 12% BisTris SDS-PAGE gel in MOPS running buffer. Bands of interest corresponding to the reactive bands on the Western blot were excised (black boxes; white = control) and in-gel tryptic digestion was performed. The peptides were identified by mass spectrometry analysis.

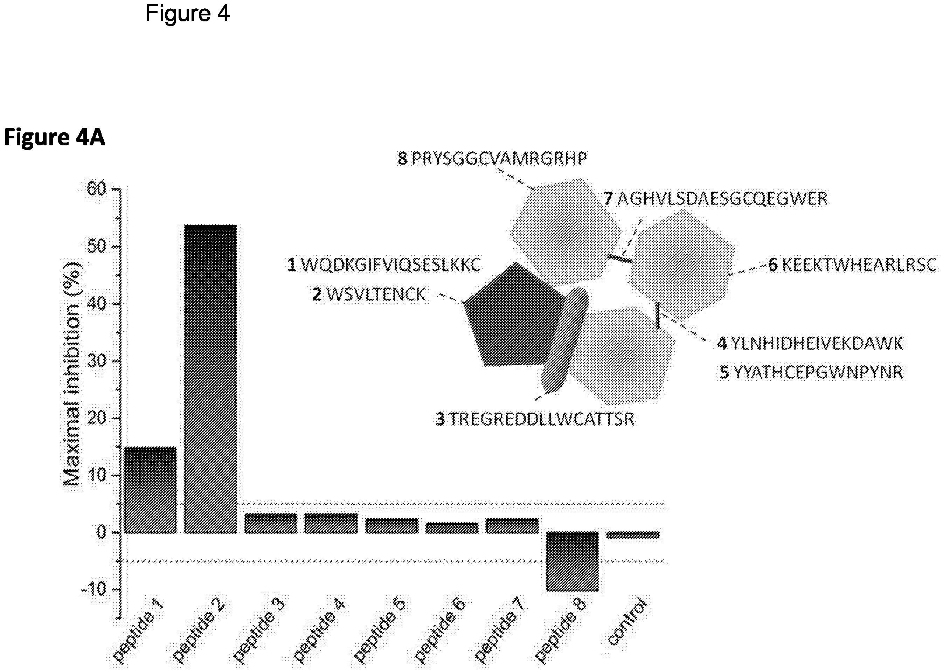

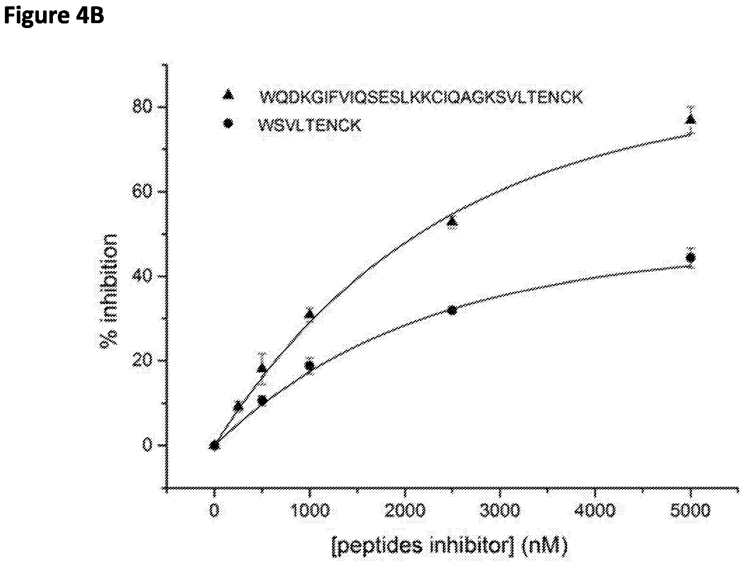

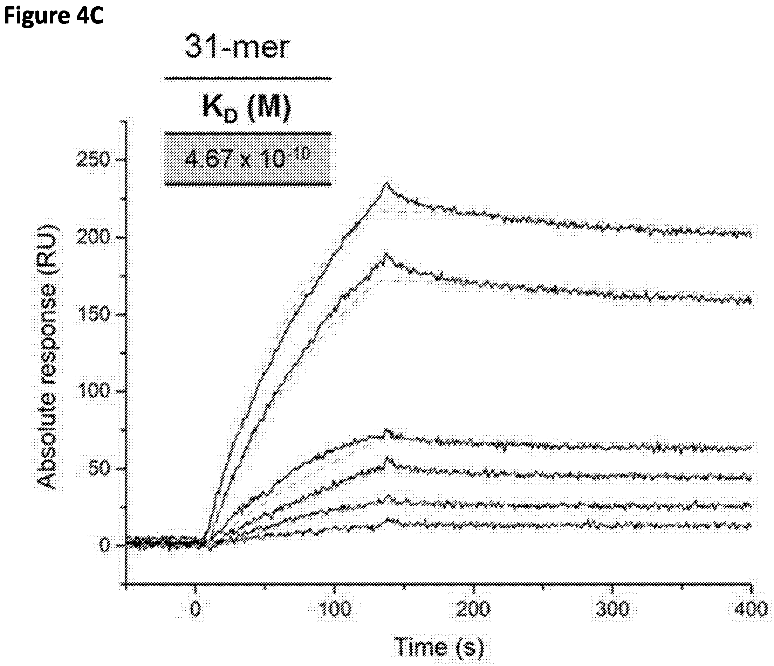

**Figure** 4 - Location of the major epitope in PLA2R. (A) Screening of inhibitory peptides identified by MS. Sequences of the synthesized peptides as well as their coverage is shown on the schematic diagram of the PLA2R fragment N-C3. Nine peptides including a control were checked for their inhibitory activity against N-C3. PLA2R-specific antibody was incubated separately with an excess of each peptide (5 µM) then injected onto the immobilized N-C3. (B) Dose-response curves of inhibition between antibody to PLA2R and N-C3 by an increasing amount of peptide 2 and 31-mer peptide. Error bars indicate SD. (C) Sensorgrams derived from injections of different concentrations of affinity purified PLA2R-specific antibody over captured 31-mer peptide. Kinetics data were fitted to a Langmuir 1:1 interaction model with mass transfer.

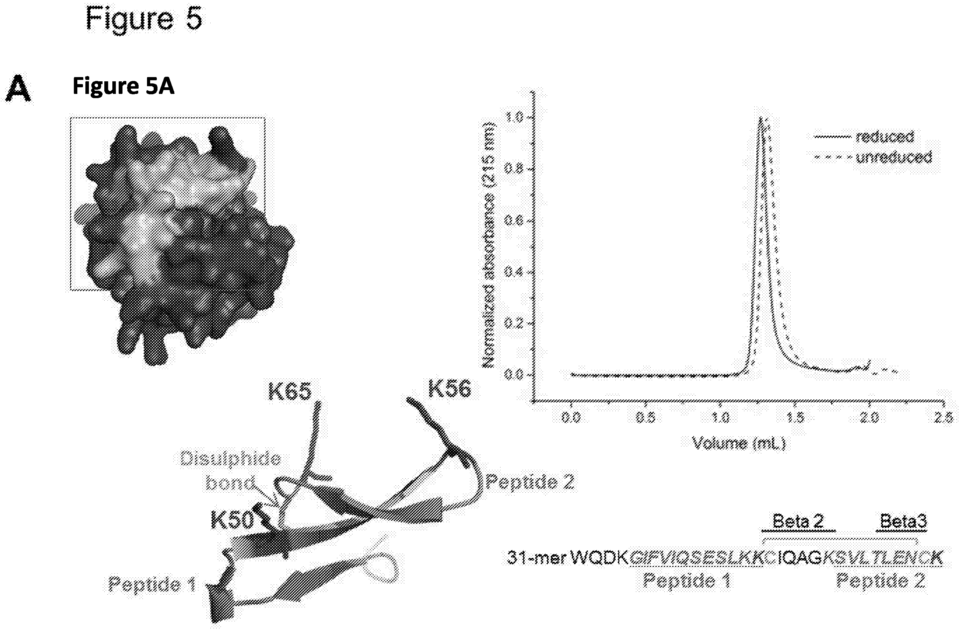

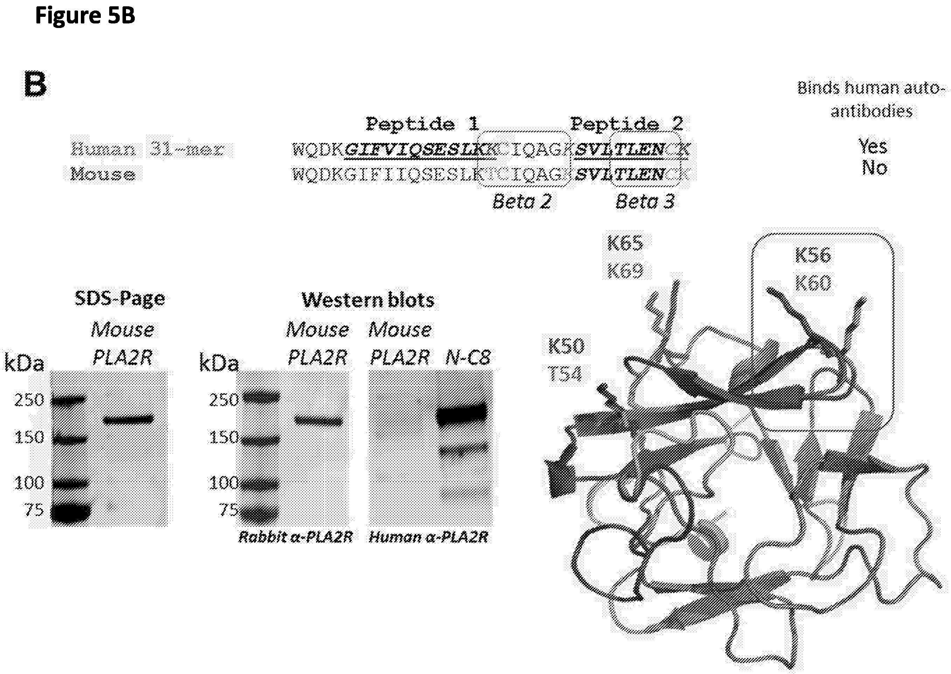

**Figure** 5 - Characterization of the dominant epitope. (A) Location of the antibody binding pocket (pink) and the predicted binding residues (red) in the ricin domain based on homology model. 3D cartoon representation of the 31-mer peptide structure comprising peptide 1 (blue) and peptide 2 (green) with the disulphide bond highlighted in orange. Inset, gel filtration elution profiles of the unreduced and reduced 31-mer peptide showing its cyclic nature in native form. (B) Western blot analysis of recombinant mouse PLA2R (R&D) and N-C8 proteins using human anti-PLA2R antibody and polyclonal rabbit anti-PLA2R antibody. Overlay of homology models for human (purple) and mouse (cyan) ricin domains showing superposition of both structures in part (dotted blue) and differences in orientation of some loops. The 31-mer peptide sequence is depicted in pink (human) and red (mouse) with key binding residues highlighted in red (human) and green (mouse). This demonstrates the different conformation of the proposed binding loop (boxed) between the two structures. Model based on PDB template d1dqga.
**Figure 6** - 3D PLA2R structure and its domains arrangement. (A) Representative area micrographs of N-C8 with highlighted images of single molecules within white squares, selected projections obtained and two-dimensional averages of the images within the corresponding class. Scale bar = 1,000 Å. (B) Electron density of N-C8 solved from ∼20,000 selected particles at 20 Å resolution and a 3D reconstruction of N-C8 showing the domains arrangement. Individual PLA2R domains were modelled using Phyre2. The domains were aligned in the EM density map using Chimera and validated using AUC hydrodynamics parameters (Table 1). Scale bar = 50 Å.
**Figure 7** - Characterization of polyclonal rabbit antibody raised against the extracellular part of PLA2R (N-C8).

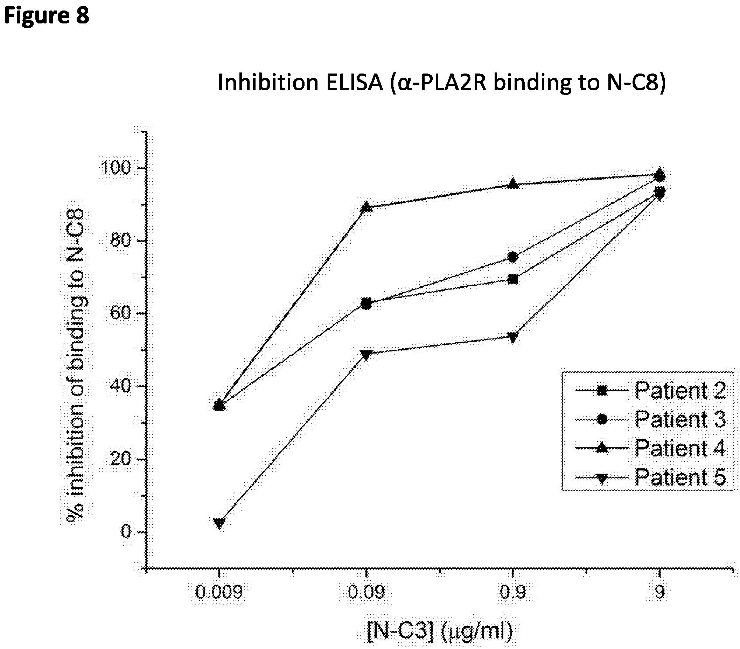

**Figure 8** - Inhibition ELISA (PLA2R-specific antibody binding to N-C8).

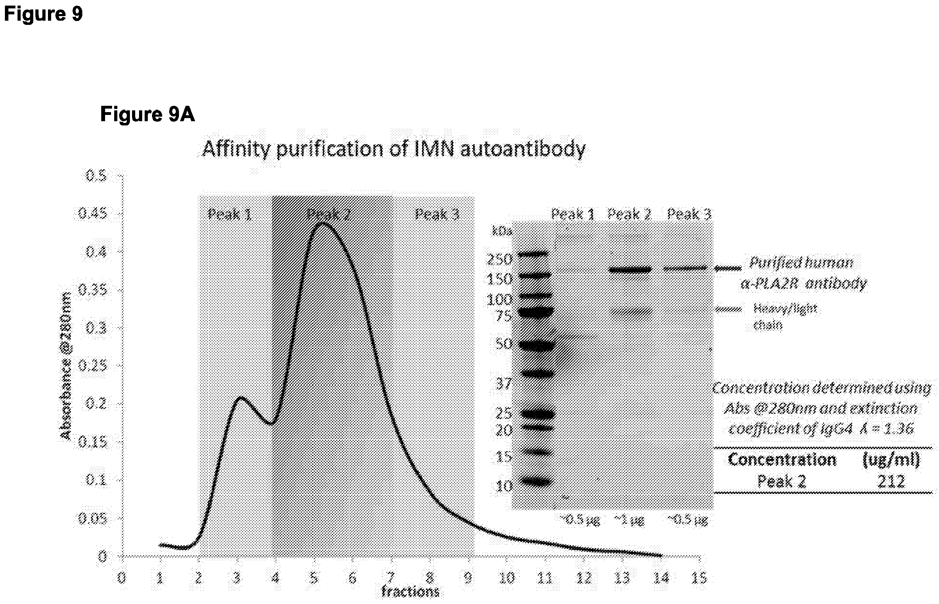

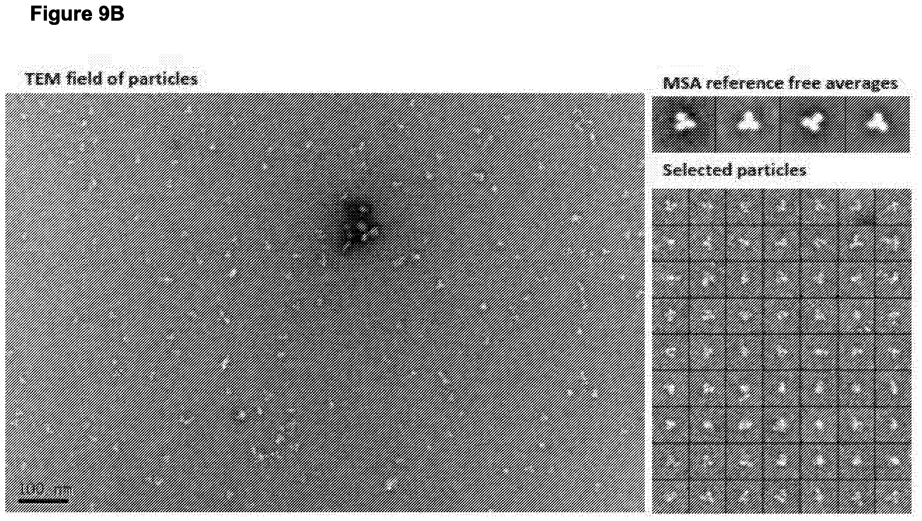

**Figure 9** - Affinity purification of IMN autoantibody.

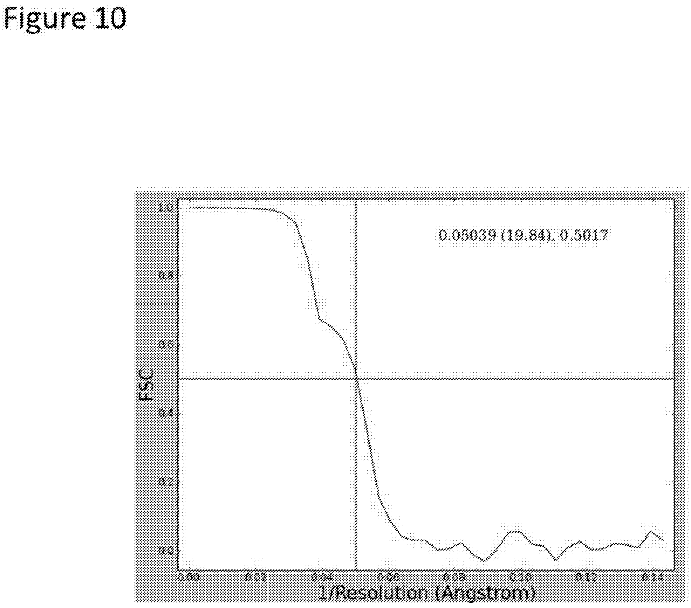

**Figure 10** **-** Estimation of the resolution of the 3D reconstructions of PLA2R N-C8.

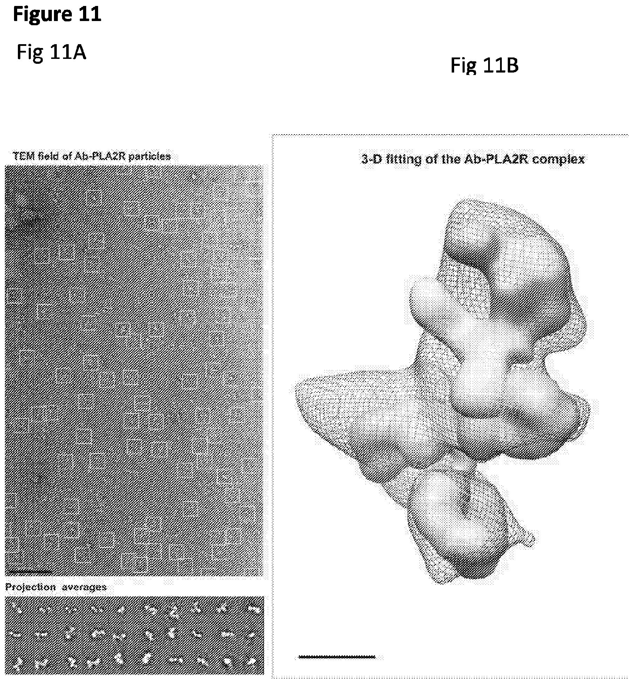

**Figure 11** - Transmission Electron Microscopy of N-C8 protein in complex with its autoantibody and a low resolution 3D structure of the complex.

### Examples

### Abstract

Phospholipase A2 receptor 1 (PLA2R) is a target antigen in 70% of patients with idiopathic membranous nephropathy (IMN). Here we describe the location and composition of the major epitope recognised by human anti-PLA2R autoantibodies which we identified in the region spanning the N-terminal cysteine-rich (CysR) ricin domain, fibronectin Type II (FNII) and the first three C-type lectin domains (CTLD) of PLA2R. This fragment inhibits antibody binding to the extracellular sequence in 90% of the human sera tested. We found the affinity between anti-PLA2R and PLA2R was five fold greater than reported affinities for anti-GBM antibodies and their target antigen. Furthermore, we showed that PLA2R conformation is sensitive to pH, although this does not influence autoantibody binding to PLA2R. To determine the composition of the epitope, we isolated eight immunoreactive tryptic fragments from recombinant extracellular PLA2R and analyzed these by mass spectrometry. The peptides were tested as inhibitors of autoantibody binding to PLA2R by surface plasmon resonance (SPR) and two peptides from the CysR ricin domain showed strong inhibition with a longer sequence covering both peptides producing 85% inhibition of autoantibody binding to PLA2R. Analysis of PLA2R, its autoantibody and the PLA2R-anti-PLA2R complex using electron microscopy and homology based representations, allowed us to generate a structural model of the major epitope and its antibody binding site. Identification of the major PLA2R epitope will enable further therapeutic advances for patients with IMN including antibody inhibition therapy and immunoadsorption of circulating autoantibodies.

### Introduction

Idiopathic membranous nephropathy (IMN) is a rare form of glomerulonephritis affecting 10-12 cases per million population. The significant discovery in 2009 that circulating antibodies to phospholipase A2 receptor 1 (PLA2R) are present in 70% of patients with IMN, identified the autoimmune nature of this pathology. Genetic evidence of the involvement of PLA2R in IMN came from the genome wide association study identifying *PLA2R* and *DQA1* as genes accountable for the genetic susceptibility to IMN. Clinical confirmation that anti-PLA2R antibodies are relevant in MN is evident from studies showing an association between high levels of anti-PLA2R and active disease, poor clinical outcome at 5 years and less chance of spontaneous remission.

In other autoimmune kidney diseases such as anti-GBM disease, which is characterized by anti-collagen IV (α3NC1) autoantibodies and ANCA vasculitis characterized by anti-MPO autoantibodies, the antigenic epitopes include both linear peptide sequences and 3D conformational structure. Knowledge of these antigen epitopes in these diseases has been important in understanding the pathological disease mechanisms and may help to classify patient subgroups and severity of disease. Differential epitope involvement in the anti-PLA2R response may also help to further classify the IMN patient phenotype. PLA2R is a member of the mannose receptor (MR) family which share a common domain structure and include the mannose receptor (CD206), Endo180, DEC-205, PLA2R and FcRY. Detailed structure based on X-ray crystallography for CD206 and conformational studies performed on FcRY have so far been used to predict the structure and function of the domains in PLA2R. However, specific experimental structural data for PLA2R is currently lacking, although this is required to improve the prospects of targeted treatments for patients with IMN.

The interactions between anti-PLA2R and PLA2R are reported to be sensitive to chemical reducing agents, stable in the detergent SDS and independent of glycosylation. However, it is unlikely that there are genetically determined amino acid changes in PLA2R specific to cases of IMN that could influence immunogenicity or contribute to a 3D conformational disease epitope. One study described linear epitopes within PLA2R using an overlapping array of short peptide sequences derived from PLA2R. Seven linear peptides were tested using an ELISA assay but no difference was seen comparing IMN seropositive patients and control sera. This suggests that 3D conformational structure is a critical feature of PLA2R epitopes. Yet, the location and nature of epitopes recognized by anti-PLA2R are unknown. This study describes a major novel PLA2R epitope bound by human anti-PLA2R autoantibodies. Our strategy was to identify the smallest fragment of extracellular PLA2R recognized by anti-PLA2R antibodies collected from a cohort of IMN patients. We analyzed this fragment by mass spectrometry-based proteomics to determine the constituent peptides. Using additional experimental data we generated a new multi-domain model of PLA2R and mapped our candidate peptides onto the model. We also investigated whether pH dependent conformational changes in the structure of the molecule was a possible contributor to the PLA2R epitope. Finally we identified the major PLA2R epitope by using candidate peptides to inhibit the binding of autoantibodies.

### Results

### Identification of the smallest immunoreactive fragment of PLA2R

We generated a series of recombinant PLA2R protein fragments including full length extracellular PLA2R (N-C8), the N-terminus to and including CTLD3 (N-C3), the N-terminus to and including CTLD2 (N-C2) and an isolated CTLD3 domain (C3). Using western blotting we compared the reactivity of rabbit anti-PLA2R (Figure 7) and human anti-PLA2R autoantibodies against these PLA2R fragments and we found that all recombinant fragments were recognized by the rabbit antibody whereas anti-PLA2R autoantibodies only recognized N-C8 and N-C3 fragments (Figure 1A). As neither N-C2 nor C3 fragments were recognized by autoantibodies, this suggests that the main epitope may be dependent on the conformational folding of domains in N-C3. To determine the ability of N-C3 to inhibit autoantibody binding, we performed an inhibition ELISA, and found the N-C3 fragment competed effectively for the binding of autoantibodies to N-C8 in approximately 90% of the patient sera tested confirming the presence of the major epitope(s) within the N-terminal to the third CTLD of the receptor (Figure 1B; Figure 8).

### The effect of pH on PLA2R conformation and epitope availability

PLA2R shares structural similarities with other members of the mannose receptor family and one member, FcRY has been shown to undergo conformational change in a pH-dependent manner. We screened a pH range from 5.0 to 7.2 and measured the associated conformational change by monitoring the thickness of the N-C8 protein layer in real time using dual polarization interferometry. This allowed us to determine a susceptible pH region around pH 6.2 where the transition in conformation occurred (Figure 1C). Both NC3 and N-C8 variants were then incubated in neutral pH 7.2 and a more acidic pH 6.2 and their sedimentation rates, which depend on the shape and size of the protein, were monitored using analytical ultracentrifugation. At pH 7.2 the N-C8 protein sedimented as a single species with a sedimentation coefficient of 8.1s (Figure 1D and Table 1). At pH 6.2 the protein became more compact which was reflected in a larger sedimentation coefficient of 9.2s. The N-C3 data (Figure 1D) collected at pH 6.2 and 7.2 yielded an identical sedimentation coefficient (5.3s) indicating that compaction of the molecule at lower pH was specific to the N-C8 receptor and not to the shorter fragment N-C3. To test whether anti-PLA2R binding to N-C8 was independent of this conformational change, single cycle kinetics between the anti-PLA2R antibody and the N-C8 receptor were performed at two pHs (pH 6.2, tight conformation and pH 7.2, extended conformation) and showed no difference in affinity (Figure 1E). This indicates that the binding of the autoantibody was not affected by altered conformation of the receptor due to the environmental pH.

### Comparison of affinity of binding to N-C3 and N-C8

We tested sera from four anti-PLA2R positive patients and showed that the autoantibody targeted the same conformational epitope as seen on the western blots of trypsinfragmented N-C3 (Figure 2A). We next determined the affinity of interaction between purified human anti-PLA2R (Figure 9) and the two variants of the PLA2R receptor (NC8 and N-C3). The autoantibodies bound with equally strong affinity of ∼0.1 nM to N-C8 and N-C3 confirming the existence of a single epitope in the N-C3 region of the protein (Figure 2B and Table 2).

The kinetic constants derived from the analysis of purified antibodies were comparable to those obtained from the patient sera, demonstrating the utility of unpurified antibodies in sera and their potential use in a diagnostic assay (Figure 2C and Table 3). Moreover, the antibody affinity was similar in all patient sera tested at approximately 0.5x10-10 M (Table 3) and higher than the apparent dissociation constant described for α3NC1 antibodies in anti-GBM disease (2x10-9 M).

### Defining the major epitope in PLA2R

To locate the major epitope, we generated immunoreactive trypsin fragments of PLA2R under non-reducing conditions having first compared trypsin digested PLA2R with native PLA2R in the standard ELISA for anti-PLA2R to confirm that the epitope was preserved in the digested antigen. Trypsin fragmentation of the N-C3 antigen, followed by multidimensional separations (OFFGEL fractionation and SDS-PAGE analysis), were performed in order to identify the smallest reactive polypeptides in the epitope. This approach allowed us to overcome the complexity of the digested sample and concentrate low abundance reactive peptides and achieve high resolution separation (Figure 3). The polypeptides of interest identified by blotting with anti-PLA2R antibody from patient sera were cut from the gel, reduced and identified by mass spectrometry (MS). MS analysis revealed eight peptides potentially constituting part of the PLA2R epitope (Table 4) and these originated from the ricin domain, FNII domain, CTLD3 domain and inter-domain loops between CTLD 1&2, CTLD 2&3.

The distribution of these peptides is discontinuous on the linear sequence but could be in proximity within the folded molecule and held together by intact disulphide bonds. These eight peptides were synthesized and we incubated the autoantibody with an excess of the candidate peptides and assessed their potential to inhibit the binding between the autoantibody and its receptor using SPR (Figure 4A). Of these peptides only peptide 1 (GIFVIQSESLKKC), representing the β1 strand and peptide 2, [(W)SVLTLENCK], forming the β3 strand from the ricin domain showed significant reduction in binding of purified autoantibody to N-C3 (Figure 4A). By extending peptide 1 towards peptide 2, it also encompasses the β2 strand to form the 31-mer (WQDKGIFVIQSESLKKCIQAGKSVLTLENCK) creating one of the 3D lobes in the beta trefoil structure. This beta 1,2,3 lobe is also maintained by a disulphide bond connecting the β2 and β3 strands (Figure 5A).9 The two main competing peptides, peptide 2 and the 31-mer peptide were titrated in a binding experiment, to compete with binding of anti-PLA2R antibody to immobilized N-C3. Peptide 2 showed reproducible inhibition up to 47% and the 31-mer peptide inhibited by 83% (Figure 4B). The competing 31-mer peptide bound to the antibody (with an affinity ∼ 5x10-10 M, Figure 4C) and this resulted in reduced affinity between this complex and N-C3 confirming the specificity of the binding. The 31-mer peptide in solution is a disulphide bonded structure illustrated by the shift in the elution profile between the reduced and unreduced state (Figure 5A). The 3D conformation of the 31-mer peptide identifies three prominent lysine residues K50, K56 and K65 with the disulphide bond between K50 and K65 (Figure 5A). The mouse PLA2R sequence which does not react with human anti-PLA2R antibodies is highly conserved across this 31-mer region except for valine V42 substituted to isoleucine I46 and the lysine K50 residue changed to threonine T54 (Figure 5B). The predicted structure of this peptide clearly shows a different conformation between the two species dependent on amino acid change at residue K50/T54 and altered K56/K60 orientation. This may explain why human autoantibodies fail to react with mouse PLA2R on western blotting (Figure 5B). To identify possible sequence homology for peptides 1&2, we used the Basic Local Alignment Search Tool (BLAST) against a database of microbial proteins and found no homology for the peptide 1 sequence. However the LTLENCK sequence of peptide 2 (SVLTLENCK) was identified in a cell wall enzyme, Dalanyl-D alanine carboxypeptidase, common in several bacterial strains including Clostridia. This finding may be relevant for pathogenesis of IMN.

### 3D PLA2R structure and arrangement of domains

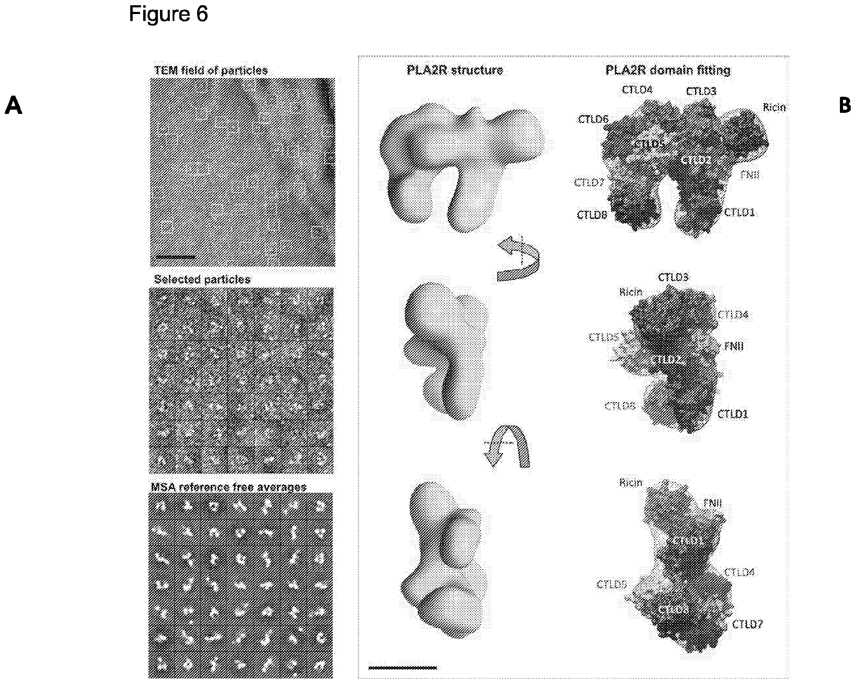

While structure for the individual domains in PLA2R can be imputed from reference X-ray crystal structures, there is no multi-domain model of PLA2R on which to map these peptide sequences. We therefore produced a 3D structure of the full length molecule N-C8 using Transmission Electron Microscopy (TEM) and single particle averaging. We established the relative positioning of the domains and as a result a predicted location of the epitope. Negative stained data was used to visualize the shape of PLA2R (Figure 6A), anti-PLA2R (Figure 9) and the preformed isolated immune complex between PLA2R and the autoantibody (Figure 11). Analysis of these datasets allowed us to generate the first 3D structure to a moderate resolution of 20 Angstroms (Figure 10) of the extracellular domains of PLA2R (Figure 6B). PLA2R is a flat structure (∼ 4 nm width) with an overall shape similar to the π symbol measuring -12 nm × 9 nm. Using homology models of the individual domains, we constructed a structural representation of the domain arrangement informed by the sharp molecular envelope produced from TEM (Figure 6B). The resulting models for both N-C3 and N-C8 were analyzed for their hydrodynamic solution properties using the program hydropro (v10). The sedimentation coefficients of the models were in good agreement with the compact form of N-C8 and the N-C3 experimental results (Table 1) giving confidence in the overall shape of the molecule. A low resolution 3D model of the immune complex confirmed the location of the anti-PLA2R binding site in the ricin domain (Figure 11).

### Discussion

We describe the location and composition of the dominant epitope in PLA2R which interacts with human autoantibodies. Located in the N-terminal CysR ricin domain, the β2-β3 strands of the trefoil structure involving the peptide WQDKGIFVIQSESLKKCIQAGKSVLTLENCK form the focus of this epitope. The crystal structure of this CysR domain is reported as a beta trefoil form also seen in ricin B,15 and is composed of twelve anti-parallel β-strands, our in each part of the three lobed structure. Three disulphide bonds have been shown to hold these lobes in place highlighting the structural importance of the disulphide bond present in the 31-mer peptide connecting β2 and β3 strands. Our data suggest that this epitope is not in the correct conformation in the small PLA2R N-C2 fragment to allow autoantibody binding. This implies an interaction between the ricin domain and the adjacent CTLD3 domain is crucial to support the correct 3D epitope structure. This requirement for cooperative conformation between adjacent domains in order to present a functional site in a protein structure has been described for the gelatin binding site in fibronectin16 and for collagen binding to the FN II domain in MR and Endo180.17 Our current best-fit model of the preformed immune complex of PLA2R and anti-PLA2R confirms that the IgG Fab binds to PLA2R in the area of the ricin/CTLD3 interaction. Current knowledge of the PLA2R sequence derived from gene sequencing suggests that the PLA2R protein structure in patients with IMN is not significantly different from that of healthy controls. Other potential mechanisms affecting antigen conformation and aberrant immune processing need exploring to understand how PLA2R becomes immunogenic. We described a pH-dependent conformational change in PLA2R (N-C8), as previously reported for FcRY and shown this is not a property of the N-C3 fragment and does not influence the affinity of antibody interaction. However, we found a critical requirement for adjacency of the ricin and CTLD3 domains, which are distant of the linear sequence but appear in a ring-like configuration in our model. This 3D conformational structure is important for epitope recognition and docking of the antibody to the target molecule. Potentially, any mechanism that controls the arrangement or interaction of the ricin and CTLD3 domains might expose or mask this epitope. This is a testable hypothesis for immunogenicity and is a significant step towards understanding the initiation of the autoimmune mechanism in IMN. Interestingly, approximately 10% of anti-PLA2R positive sera react variably to another epitope not in N-C3 fragment and which by implication is in C4-C8 region of PLA2R. Further work is needed to identify the nature of this second epitope and whether antibodies to this minor epitope are associated with a particular clinical phenotype of IMN. The affinity of anti-PLA2R antibodies in different patient sera is uniformly high and approximately five fold greater than that described for the classic autoantibody anti-collagen IV (α3NC1). This could explain the clinical paradox of PLA2R positive biopsies associated with absence of anti-PLA2R antibodies in the serum. We suggest that as anti-PLA2R antibodies are secreted by plasma cells, the high affinity of the antibody will ensure the autoantibody is adsorbed out of serum onto PLA2R positive cells such as podocytes until the rate of production of antibody exceeds the rate of removal from the serum, at which point there will be free detectable circulating anti-PLA2R. These biopsy positive, seronegative cases may, therefore, represent an early phase of the immunological stage of disease. Having identified the β2-β3 strands (31-mer peptide) containing an inherent disulphide bond, we mapped this peptide to the reported ricin domain structure and noted the formation of a surface charge patch on the domain. Specifically three lysines K50, K56 and K65 are brought together with K50 and K65 linked via the disulphide bond. It will be important to further investigate how the availability of this binding pocket to autoantibodies is controlled by adjacent domains. The natural variation in this 31-mer PLA2R amino acid sequence between man and mouse may explain why autoantibodies fail to bind to mouse PLA2R. It has been proposed that the induction of autoimmunity may be mechanistically driven by infection whereby, an immune response against bacterial or viral agent cross reacts with a self-component. In this regard, preliminary bioinformatic analysis of peptide 2 in BLAST searches against microbial protein databases revealed complete homology for the sequence LTLENCK which is part of the bacterial cell wall enzyme D-alanyl-D alanine carboxypeptidase common to Clostridium and other bacteria. Clonal expansion of B cells driven by common infections may allow MN genetically-susceptible individuals to bind PLA2R through the B cell receptor, ingest, degrade and present PLA2R peptides on Class II receptors (DQA1) to engage T cell help and promote autoantibody production. In autoimmune mechanisms over time, there is often a change in use of epitopes on the autoantigen, in some cases as proposed for anti-GBM antibodies, the binding of the autoantibody induces neo-epitopes initiating "epitope spreading." Now that we have defined the major epitope, we can investigate whether there is change in use of this epitope over time in this chronic disease, from initiation and first presentation of disease through induction of remission, relapse and in recurrent disease post transplantation. Knowledge of the PLA2R epitope is potentially useful in developing novel therapies for translation into the clinic for patient benefit. The use of the 31-mer peptide as an inhibitor of anti-PLA2R binding to the podocyte may modulate the consequences of anti-PLA2R mediated cell biology. Alternatively, the 31-mer peptide coupled to immunoadsorption columns could provide an effective means of removing anti-PLA2R particularly for patients resistant to standard main line immunosuppressive drugs.

### Concise Methods

### Cloning, expression and purification of PLA2R N-C8, N-C3, N-C2 and C3

The previously described5 codon-optimized clone of human extracellular PLA2R (N-C8) was modified to generate the smaller PLA2R fragments expression vectors. The resulting constructs were transfected into HEK 293-EBNA1 cells (human embryonic kidney cells; Invitrogen) using Lipofectamine 2000 reagent (Invitrogen). The secreted proteins were purified using nickel affinity chromatography. Conditioned medium containing 20mM imidazole was loaded onto a 5ml HiTrap Excell nickel affinity chromatography column (GE Healthcare). Bound proteins were eluted by using a linear gradient of 20-500 mM imidazole over 10 column volumes in a buffer containing 10 mM BisTris, 150 mM NaCl pH 7.2. The PLA2R containing fractions were desalted using PD-10 columns (GE Healthcare) into 10 mM BisTris pH 7.2 and further purified by anion exchange chromatography on a MiniQ column using the Ettan purifier HPLC system (GE Healthcare). The proteins were eluted by using a linear gradient of 0-1 M NaCl. Buffer conditions were optimized using an Optim screening instrument (Avacta).

### N-C3 Inhibition ELISA

The ELISA method was a variation on the standard assay previously described. Briefly, PLA2R (N-C8) at 125 ng ml-1 was coated onto ELISA plates overnight and plates blocked with Superblock (Thermo Scientific). 50 µl of 1:100 dilution of patient serum plus 50 µl of either buffer or NC-3 fragment to give a final concentration in the well of 9.0 µg ml-1 , 0.9 µg ml-1 , 0.09 µg ml-1 and 0.009 µg ml-1 were preincubated for 2 hours at room temperature before adding to the ELISA plate for a further 2 hours with shaking. Following extensive washing, anti-human IgG-HRP at 1:25,000 dilution in assay buffer was added to all wells for 2 hour incubation. Following washing, the plate was developed by adding substrate and the reaction terminated at 5 minutes by addition of 1 M H2SO4. Plates were read at 450 nm and inhibition curves constructed. The concentration of 9.0 µg ml-1 was used to compare the inhibition profile of 43 different sera previous reported as anti-PLA2R positive.

### Dual polarisation interferometry

Dual polarisation interferometry was used to provide quantitative information on the thickness, the refractive index and density of the immobilized PLA2R layer with the capability to measure conformational change in real time. The DPI experiments were performed on a Farfield AnaLight instrument. Purified recombinant N-C8 and N-C3 proteins (at a concentration of 50 µg ml-1) were immobilized onto an amine functionalized AnaChip using sulfo-GMBS (Pierce) an amine-to-sulfhydryl crosslinker (2 mg ml-1). After stabilizing the immobilized N-C8 and N-C3 with sufficient rinsing with BisTris running buffer (10 mM BisTris, pH 7.2, 150 mM NaCl), the proteins were then exposed to a decreasing pH range (pH 7.2, 6.8, 6.4, 6.2, 6, 5.9, 5.8, 5.5 and 5). N-C8 and N-C3 were regenerated between the ifferent pHs with running buffer pH 7.2. Analysis of the raw data and determination of density, thickness and mass per unit area was achieved using the *Ana*Light® Bio200 analysis software (Farfield Scientific).

### Structure analysis by sedimentation velocity

The sedimentation coefficients for recombinant N-C8 and N-C3 proteins (at 150 µg ml-1) were determined from velocity experiments using the Optima XL-A ultracentrifuge (Beckman Instruments).The experiments were performed using double sector cells and a rotor speed of 74,500g for N-C8 and 116,500g for N-C3, taking 150 scans at 1.5 minutes intervals at a wavelength of 280 nm and at a temperature of 20°C. The sedimenting boundaries were analyzed using the program Sedfit v8.7.

### Binding studies using Surface Plasmon Resonance

Purified recombinant N-C8 or N-C3 proteins (100 µg ml-1) diluted in 10 mM sodium acetate pH 4.5 were immobilized on a ProteOn GLM sensor chip using standard amine coupling chemistry. Ligand densities of 6,000 RU and 5,000 RU respectively were achieved. The first set of kinetic runs were performed using 6 concentrations of purified anti-PLA2R antibody as analytes (1, 2, 5, 10, 15 and 20 nM) in HEPES buffer pH 7.2 containing 0.005% Tween 20. Each injection lasted 120 s at a flow rate of 80 µl ml-1 with a 900 s dissociation phase. Tightly bound proteins were then dissociated by two successive (40 s) injections of 100 mM glycine pH 2.2. The second series of kinetic used patient sera. Seven dilutions of sera containing anti-PLA2R antibody (1/100, 1/200, 1/500, 1/800, 1/1,000, 1/1,500 and 1/2,000) were injected onto the same immobilized N-C8 and N-C3 proteins. Samples were all double referenced with blank row (injected with running buffer) and control serum. The data were analyzed and fitted to a Langmuir 1:1 interaction model. Single cycle kinetics between immobilized purified anti-PLA2R antibody (2,000 RU bound onto a CM5 sensor chip) and C8 were performed on a BIAcore T200 instrument (BIAcore AB). Two cycles of 1, 2, 5, 10 and 15 nM of N-C8 were injected, one at pH 6.2 and the other at pH 7.2. Kinetic parameters were determined with the manufacturer's BIAevaluation 4.1 software. Peptide inhibition assays were carried out as follows. Each individual peptide (5 µM) was incubated with 10 nM of purified anti-PLA2R antibody for 1 hour then the complex was injected on the captured N-C3 (5,000 RU bound). Peptides 2 and 31-mer peptide were titrated to compete the binding between anti-PLA2R antibody and N-C3 with a range of 0, 0.25, 0.75, 1, 2, 3 and 5 µM peptides.

### Determination of anti-PLA2R antibody concentration in patient sera

Quantitation of serum antibody concentrations was performed on the Proteon XPR-36 (Bio-Rad) SPR instrument where the initial binding rates of purified human anti-PLA2R binding to N-C3 were used as standard curve to determine the concentration of anti-PLA2R in the unknown serum samples.

### OFFGEL fractionation and Mass spectrometry analysis

N-C8 and N-C3 purified proteins (300 µg) were digested with 15 µg proteomics grade trypsin (Sigma) overnight at 37°C. Polypeptides (unreduced) were separated by isoelectric point and size and the smallest fragments were identified by western blotting with patient sera. Bands of interest corresponding to the reactive bands on the Western blot were excised and in-gel tryptic digestion was performed. Dried gel pieces were reduced with 10 mM dithiothreitol and alkylated with 55 mM iodoacetamide. Digested samples were analysed by LC-MS/MS using an UltiMate® 3,000 Rapid Separation LC (RSLC, Dionex Corporation) coupled to a LTQ Velos Pro (Thermo Fisher Scientific) mass spectrometer. Data produced were searched using Mascot (Matrix Science UK), against the Uniprot_human database version 2011_05_03. Data were validated using Scaffold (Proteome Software).

### Negatively stained Transmission Electron Microscopy (TEM)

Negatively stained samples of PLA2R, antibody or the PLA2R-antibody complex (10-20 µg/ml) were absorbed to the surface of a freshly glow-discharged 400 mesh carbon coated grid (EMS) for 30 s, washed twice in 10 µl droplets of distilled water and then placed on a 10 µl droplet of 5% (w/v) uranyl acetate for 30 s and air dried. Low dose data (10-20 e-/angstrom) were recorded on a G30 Polara operating at 200 kV and a nominal magnification of 39,000x with a Gatan 4K CCD camera. CCD images were recorded with a 1 s exposure at defocus values between 0.5 - 2.5 µm and at a sampling increment of 3.05 Å/pixel.

### Single particle analysis and 3D structure determination

Single particle averaging was performed using EMAN2.22 Data-sets were selected using a combination of manual and semi-automated swarm picking. Following full CTF correction, each dataset was subjected to MSA 2D classification using cross correlation coefficient (CCC) as the main comparator. A selection of uniquely positioned projection averages were selected and used to generate an initial 3D model for refinement (C1 symmetry was applied to the processing for all datasets). This model was used as a start model for 8 rounds of iterative refinement, using FRC as the main alignment comparator, to produce a selfconsistent 3D structure which was low pass Gaussian filtered to 20 Å resolution. Resolution estimates of the final structures were determined using the Fourier Shell Correlation using a cut-off value of 0.5. 3D volumes were examined and modelling was performed using Chimera.

### Modelling

Homology models of the domains were generated using the software Phyre2. These were assembled into the EM density map using Chimera v1.8 without the interglobular linkers present. The resulting models of both the N-C3 and N-C8 were converted to shell models using the program Hydropro10. The hydrodynamic outputs were compared to experimental data for the hydrodynamic radius deduced from light scattering, and sedimentation coefficient determined using analytical ultracentrifugation, in order to establish the best arrangement of the domains.

### Tables

**Table 1 Hydrodynamic parameters determined for N-C8 and N-C3 at pH 7.4 and pH 6.2.**

| | **Theoretical mass (Da)** | **Mass MALLS (Da)^{#}** | **Sedimentation coefficient (S_{20,W})** | **Frictional ratio (f/f₀)*** | **Hydrodynamic radius (nm)**** |
|---|---|---|---|---|---|
| **N-C3 pH 7.4** | 79,461 | 88,820 | 5.29 ± 0.09 | 1.38 | 3.81 ± 0.23 |
| **N-C3 pH 6.2** | 79,461 | N/D | 5.35 ± 0.10 | 1.39 | N/D |
| **N-C8 pH 7.4** | 164,308 | 188,800 | 8.08 ± 0.21 | 1.51 | 6.25 ± 0.19 |
| **N-C8 pH 6.2** | 164,308 | N/D | 9.23 ± 0.25 | 1.32 | N/D |

| | | | | | |
|---|---|---|---|---|---|
| ^{#} calculated with a *dn*/*dc* of 0.18 ml g⁻¹ **f*/*f₀* from Mass derived from MALLS ** hydrodynamic radius from QELS | | | | | |

Density 1.0045 g ml⁻¹

Viscosity 0.01017 poise

*Vbar* 0.726 N-C3, 0.727 N-C8

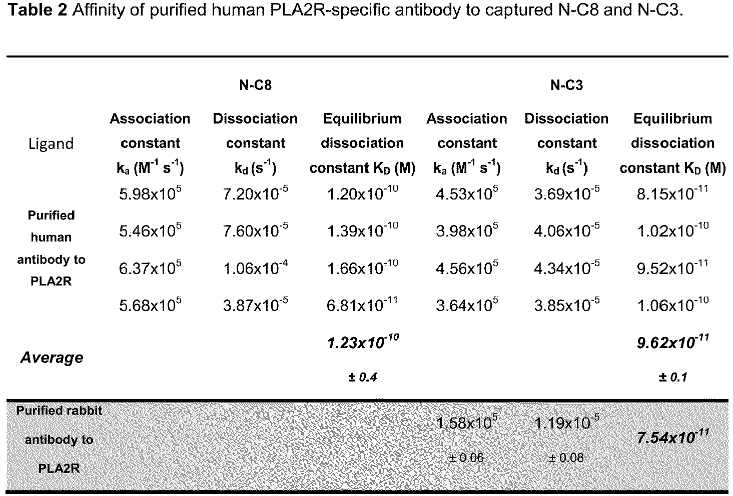

This table summarizes the kinetic constants of 4 separate runs for the binding of purified human PLA2R-specific antibody to N-C8 and N-C3. Results were obtained after reference subtraction. The association (kₐ), dissociation (k_{d}) and equilibrium (K_{D}) constants for each runs were similar and revealed an overall high binding affinity with an apparent K_{D} of -0.1 nM.

**Table 3 Concentrations and affinity of antibodies to PLA2R.**

| **Patients** | **ELISA (U ml⁻¹**) | **Concentration (µM)** | **K_{D} (M)** |
|---|---|---|---|
| **1** | 30,000 | 1.4 ± 0.06 | 0.49 × 10⁻¹⁰ |
| **2** | 2,600 | 0.32 ± 0.1 | 0.45 × 10⁻¹⁰ |
| **3** | 30,000 | 1.0 ± 0.12 | 0.43 × 10⁻¹⁰ |
| **4** | 2,300 | 0.36 ± 0.19 | 0.85 × 10⁻¹⁰ |
| **5** | 9,300 | 0.51 ± 0.18 | 0.32 × 10⁻¹⁰ |

Absolute concentrations of PLA2R-specific antibody detected in 5 patient sera tested by ELISA (U ml⁻¹) and derived from 3 kinetic runs (µM). The last column is the kinetic constants K_{D} for the binding of patient autoantibodies to immobilized N-C3.

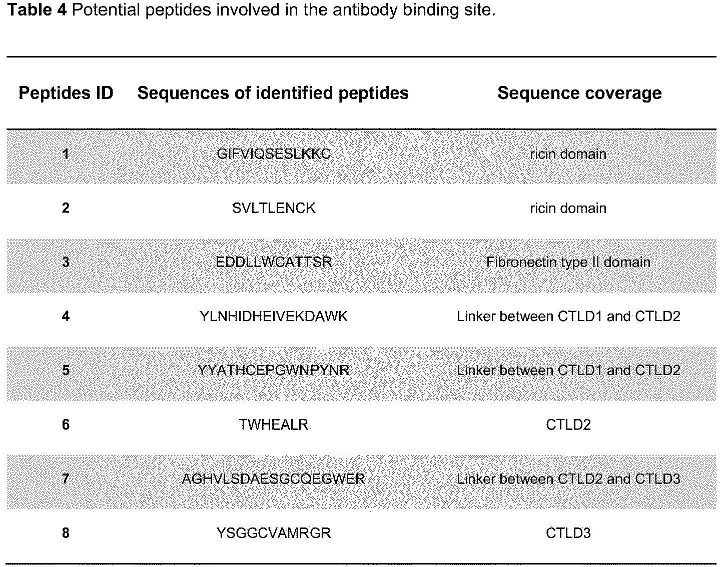

List generated from the mass spectrometry analysis of the potential peptides involved in the antibody binding site. Sequences of the identified peptides as well as their coverage are described in the table.

### Sequence information

**SEQ ID NO:1 ― Amino acid sequence of full-length human PLA2R** 
10 20 30 40 50 60
MLLSPSLLLL LLLGAPRGCA EGVAAALTPE RLLEWQDKGI FVIQSESLKK CIQAGKSVLT

70 80 90 100 110 120
LENCKQANKH MLWKWVSNHG LFNIGGSGCL GLNFSAPEQP LSLYECDSTL VSLRWRCNRK

130 140 150 160 170 180
MITGPLQYSV QVAHDNTVVA SRKYIHKWIS YGSGGGDICE YLHKDLHTIK GNTHGMPCMF

190 200 210 220 230 240
PFQYNHQWHH ECTREGREDD LLWCATTSRY ERDEKWGFCP DPTSAEVGCD TIWEKDLNSH

250 260 270 280 290 300
ICYQFNLLSS LSWSEAHSSC QMQGGTLLSI TDETEENFIR EHMSSKTVEV WMGLNQLDEH

310 320 330 340 350 360
AGWQWSDGTP LNYLNWSPEV NFEPFVEDHC GTFSSFMPSA WRSRDCESTL PYICKKYLNH

370 380 390 400 410 420
IDHEIVEKDA WKYYATHCEP GWNPYNRNCY KLQKEEKTWH EALRSCQADN SALIDITSLA

430 440 450 460 470 480
EVEFLVTLLG DENASETWIG LSSNKIPVSF EWSNDSSVIF TNWHTLEPHI FPNRSQLCVS

490 500 510 520 530 540
AEQSEGHWKV KNCEERLFYI CKKAGHVLSD AESGCQEGWE RHGGFCYKID TVLRSFDQAS

550 560 570 580 590 600
SGYYCPPALV TITNRFEQAF ITSLISSVVK MKDSYFWIAL QDQNDTGEYT WKPVGQKPEP

610 620 630 640 650 660
VQYTHWNTHQ PRYSGGCVAM RGRHPLGRWE VKHCRHFKAM SLCKQPVENQ EKAEYEERWP
 
670 680 690 700 710 720
FHPCYLDWES EPGLASCFKV FHSEKVLMKR TWREAEAFCE EFGAHLASFA HIEEENFVNE

730 740 750 760 770 780
LLHSKFNWTE ERQFWIGFNK RNPLNAGSWE WSDRTPVVSS FLDNTYFGED ARNCAVYKAN

790 800 810 820 830 840
KTLLPLHCGS KREWICKIPR DVKPKIPFWY QYDVPWLFYQ DAEYLFHTFA SEWLNFEFVC

850 860 870 880 890 900
SWLHSDLLTI HSAHEQEFIH SKIKALSKYG ASWWIGLQEE RANDEFRWRD GTPVIYQNWD

910 920 930 940 950 960
TGRERTVNNQ SQRCGFISSI TGLWGSEECS VSMPSICKRK KVWLIEKKKD TPKQHGTCPK

970 980 990 1000 1010 1020
GWLYFNYKCL LLNIPKDPSS WKNWTHAQHF CAEEGGTLVA IESEVEQAFI TMNLFGQTTS

1030 1040 1050 1060 1070 1080
VWIGLQNDDY ETWLNGKPVV YSNWSPFDII NIPSHNTTEV QKHIPLCALL SSNPNFHFTG

1090 1100 1110 1120 1130 1140
KWYFEDCGKE GYGFVCEKMQ DTSGHGVNTS DMYPMPNTLE YGNRTYKIIN ANMTWYAAIK

1150 1160 1170 1180 1190 1200
TCLMHKAQLV SITDQYHQSF LTVVLNRLGY AHWIGLFTTD NGLNFDWSDG TKSSFTFWKD

1210 1220 1230 1240 1250 1260
EESSLLGDCV FADSNGRWHS TACESFLQGA ICHVPPETRQ SEHPELCSET SIPWIKFKSN

1270 1280 1290 1300 1310 1320
CYSFSTVLDS MSFEAAHEFC KKEGSNLLTI KDEAENAFLL EELFAFGSSV QMVWLNAQFD

1330 1340 1350 1360 1370 1380
GNNETIKWFD GTPTDQSNWG IRKPDTDYFK PHHCVALRIP EGLWQLSPCQ EKKGFICKME

1390 1400 1410 1420 1430 1440
ADIHTAEALP EKGPSHSIIP LAVVLTLIVI VAICTLSFCI YKHNGGFFRR LAGFRNPYYP
 
1450 1460
ATNFSTVYLE ENILISDLEK SDQ
**SEQ ID NO:2**
   K-X1-X2-X3-X4-X5-K-X6-X7-X8-X9-X10-X11-X12-X13-K
SEQ ID NO:3
   KCIQAGKSVLTLENCK
SEQ ID NO:4
   WQDKGIFVIQSESLKKCIQAGKSVLTLENCK
SEQ ID NO:5
   GIFVIQSESLKK

## Claims

1. A peptide consisting of a length of up to 40 amino acid residues, the peptide comprising an amino acid sequence K-X1-X2-X3-X4-X5-K-X6-X7-X8-X9-X10-X11-X12-X13-K (SEQ ID NO:2), wherein X1 and X13 are both cysteine residues and wherein the amino acid sequence set forth in SEQ ID NO:2 shares at least 80% identity with SEQ ID NO: 3 over the entire length of the sequence.

2. A peptide according to any preceding claim having a length of 31 amino acid residues.

3. A peptide according to claim 1 or claim 2: wherein the peptide:
i) comprises additional amino acid residues located at the N terminal of SEQ ID NO:2;
ii) comprises a fragment of PLA2R comprising the sequence from amino acid residues 50 to 65 of the full-length protein (SEQ ID NO:3);
iii) consists of SEQ ID NO:3;
iv) comprises or consists of a fragment of PLA2R comprising the sequence from amino acid residues 35 to 65 of the full-length protein (SEQ ID NO:4);
v) comprises at least one further amino acid sequence from the group consisting of: a sequence from the Fibronectin II domain of PLA2R; a sequence from a CTLD domain of PLA2R, such as a sequence from the CTLD1 domain of PLA2R, a sequence from the CTLD2 domain of PLA2R, and a sequence from the CTLD3 domain of PLA2R,
optionally wherein:
i) the sequence from the Fibronectin II domain of PLA2R comprises the sequence EDDLLWCATTSR; or
ii) the sequence from the CTLD domain of PLA2R comprises a sequence selected from the group consisting of: YLNHIDHEIVEKDAWK; YYATHCEPGWNPYNR; TWHEALR; AGHVLSDAESGCQEGWER; and YSGGCVAMRGR;
vi) shares at least 80% identity with a SEQ ID NO:4.

4. A peptide according to any of claims 1 to 3 for use in the prevention or treatment of kidney disease.

5. A pharmaceutical composition comprising a peptide according to any of claims 1 to 3 and a pharmaceutically acceptable carrier.

6. A device for extracorporeal treatment of a patient's blood, the device comprising:
• a peptide according to any of claims 1 to 3; and
• means for separating the peptide from blood.

7. A device according to claim 6, wherein:
i) the means for separating the binding partner from blood comprises a substrate of a material selected from the group consisting of: cellulose; cellulose derivatives; agarose; agarose derivatives; polysulphone; polysulphone derivatives; polyacrylamide; polyacrylamide derivatives; and nylon; and/or
ii) the device is provided in a form selected from the group consisting of: hollow fibre cassettes, membranes, and beads.

8. A peptide for use in accordance with claim 4 for use in the treatment or prevention of kidney disease, wherein the kidney disease is selected from the group consisting of: primary renal failure; membranous nephropathy, such as idiopathic membranous nephropathy or de novo membranous nephropathy; and focal segmental glomerulosclerosis.

9. A method of determining levels of anti-PLA2R antibodies in a subject, the method comprising:
• contacting a peptide according to any of claims 1 to 3 with a sample of a body fluid from the subject, such that anti-PLA2R antibodies present in the subject's body fluid sample are able to bind to and be retained by the peptide; and
• determining the amount of anti-PLA2R antibodies retained by the peptide.

10. A method according to claim 9 for use in diagnosing kidney disease in a subject, the method comprising:
• determining *in vitro* the amount of anti-PLA2R antibodies in a body fluid sample from a subject in a method according to claim 9; and
• comparing the determined amount with a reference value,
wherein if the determined amount of anti-PLA2R antibodies in the sample is equal to or greater than the reference value, this indicates that the subject has kidney disease.

11. A method according to claim 10, wherein the subject is one that is considered to be at risk of kidney disease.

12. A method according to claim 9 for use in selecting a suitable regimen for the prevention or treatment of kidney disease, the method comprising:
• determining the amount of anti-PLA2R antibodies in a body fluid sample from a subject in a method according to claim 9; and
• comparing the determined amount with a reference value,
wherein if the determined amount of anti-PLA2R antibodies in the sample is equal to or greater than the reference value, this indicates that the subject will benefit from a regimen for prevention or treatment of kidney disease utilising a binding partner for an anti-PLA2R antibody.

13. A method according to claim 12, wherein:
i) the subject is an individual diagnosed as having kidney disease, where the cause of the kidney disease has not been determined; and/or
ii) the method further comprises obtaining a value representative of the amount of anti-PLA2R antibodies in the subject's body fluid, and comparing this obtained value with a reference value, wherein if the obtained value is larger than the reference value this indicates that the subject will benefit from a regimen for prevention or treatment of kidney disease utilising a peptide according to any of claims 1 to 3.

14. A method according to claim 9 for use in monitoring effectiveness in a subject of a treatment regimen for prevention or treatment of kidney disease, the method comprising:
• assaying a sample of a body fluid of a subject undergoing a treatment regimen for prevention or treatment of kidney disease, to determine the amount of anti-PLA2R antibodies present in the subject's body fluid sample by a method according to claim 9;
• comparing the determined amount with a reference value; and
• based on this comparison, determining whether the subject's treatment regimen is effective for prevention or treatment of kidney disease.

15. A method according to claim 14, wherein:
i) the reference value is the mean plus three standard deviations of the amount of anti-PLA2R antibody in approximately 30 samples from normal healthy individuals without kidney disease or the reference value is representative of the amount of anti- PLA2R antibodies present in a comparable sample from the subject at an earlier time point;
ii) wherein the subject is a patient undergoing therapy for kidney disease;
or
iii) wherein the subject is a participant in a clinical trial.

## Patentansprüche

1. Peptid, bestehend aus einer Länge von bis zu 40 Aminosäureresten, wobei das Peptid eine Aminosäuresequenz K-X1-X2-X3-X4-X5-K-X6-X7-X8-X9-X10-X11-X12-X13-K (SEQ ID NO:2) umfasst, wobei X1 und X13 beide Cysteinreste sind und wobei die Aminosäuresequenz festgelegt in SEQ ID NO:2 zumindest 80 % Identität mit SEQ ID NO: 3 über die gesamte Länge der Sequenz teilt.

2. Peptid nach einem vorhergehenden Anspruch, eine Länge von 31 Aminosäureresten aufweisend.

3. Peptid nach Anspruch 1 oder Anspruch 2: wobei das Peptid:
i) zusätzliche Aminosäurereste umfasst, die sich an dem N-Terminus von SEQ ID NO:2 befinden;
ii) ein Fragment von PLA2R umfasst, das die Sequenz der Aminosäurereste 50 bis 65 des Proteins voller Länge umfasst (SEQ ID NO: 3);
iii) aus SEQ ID NO: 3 besteht;
iv) ein Fragment von PLA2R umfasst oder daraus besteht, umfassend die Sequenz der Aminosäurereste 35 bis 65 des Proteins voller Länge (SEQ ID NO: 4);
v) zumindest eine weitere Aminosäuresequenz aus der Gruppe bestehend aus Folgendem umfasst: einer Sequenz aus der Fibronectin-II-Domäne von PLA2R; einer Sequenz aus einer CTLD-Domäne von PLA2R, wie einer Sequenz aus der CTLD1-Domäne von PLA2R, einer Sequenz aus der CTLD2-Domäne von PLA2R und einer Sequenz aus der CTLD3-Domäne von PLA2R,
wobei optional:
i) die Sequenz aus der Fibronectin-II-Domäne von PLA2R die Sequenz EDDLLWCATTSR umfasst; oder
ii) die Sequenz aus der CTLD-Domäne von PLA2R eine Sequenz ausgewählt aus der Gruppe bestehend aus Folgendem umfasst: YLNHIDHEIVEKDAWK; YYATHCEPGWNPYNR; TWHEALR; AGHVLSDAESGCQEGWER; und YSGGCVAMRGR;
vi) zumindest 80 % Identität mit einer SEQ ID NO: 4 teilt.

4. Peptid nach einem der Ansprüche 1 bis 3 zur Verwendung bei der Prävention oder Behandlung von Nierenerkrankung.

5. Pharmazeutische Zusammensetzung, umfassend ein Peptid nach einem der Ansprüche 1 bis 3 und einen pharmazeutisch annehmbaren Träger.

6. Vorrichtung zur extrakorporalen Behandlung von Blut eines Patienten, wobei die Vorrichtung Folgendes umfasst:
• ein Peptid nach einem der Ansprüche 1 bis 3; und
• Mittel zum Trennen des Peptids von Blut.

7. Vorrichtung nach Anspruch 6, wobei:
i) das Mittel zum Trennen des Bindungspartners von Blut ein Substrat aus einem Material ausgewählt aus der Gruppe bestehend aus Folgendem umfasst: Cellulose; Cellulosederivaten; Agarose; Agarosederivaten; Polysulfon; Polysulfonderivaten; Polyacrylamid; Polyacrylamidderivaten; und Nylon; und/oder
ii) die Vorrichtung in einer Form bereitgestellt ist, ausgewählt aus der Gruppe bestehend aus: Hohlfaserkassetten, Membranen und Kügelchen.

8. Peptid zur Verwendung nach Anspruch 4 zur Verwendung bei der Behandlung oder Prävention von Nierenerkrankung, wobei die Nierenerkrankung ausgewählt ist aus der Gruppe bestehend aus: primärem Nierenversagen; membranöser Nephropathie, wie idiopathischer membranöser Nephropathie oder membranöser de-novo-Nephropathie; und fokaler segmentaler Glomerulosklerose.

9. Verfahren zum Bestimmen der Spiegel von Anti-PLA2R-Antikörpern in einem Subjekt, wobei das Verfahren Folgendes umfasst:
• Kontaktieren eines Peptids nach einem der Ansprüche 1 bis 3 mit einer Probe einer Körperflüssigkeit des Subjekts, sodass Anti-PLA2R-Antikörper, die in der Körperflüssigkeitsprobe des Subjekts vorhanden sind, an das Peptid binden und von diesem zurückgehalten werden können; und
• Bestimmen der Menge an Anti-PLA2R-Antikörpern, die von dem Peptid zurückgehalten werden.

10. Verfahren nach Anspruch 9 zur Verwendung beim Diagnostizieren einer Nierenerkrankung bei einem Subjekt, wobei das Verfahren Folgendes umfasst:
• in-vitro-Bestimmen der Menge an Anti-PLA2R-Antikörpern in einer Körperflüssigkeitsprobe eines Subjekts in einem Verfahren nach Anspruch 9; und
• Vergleichen der bestimmten Menge mit einem Referenzwert,
wobei, wenn die bestimmte Menge an Anti-PLA2R-Antikörpern in der Probe gleich dem oder größer als der Referenzwert ist, dies angibt, dass das Subjekt Nierenerkrankung aufweist.

11. Verfahren nach Anspruch 10, wobei das Subjekt eines ist, von dem angenommen wird, dass es für Nierenerkrankung gefährdet ist.

12. Verfahren nach Anspruch 9 zur Verwendung beim Auswählen eines geeigneten Schemas zur Prävention oder Behandlung von Nierenerkrankung, wobei das Verfahren Folgendes umfasst:
• Bestimmen der Menge an Anti-PLA2R-Antikörpern in einer Körperflüssigkeitsprobe eines Subjekts in einem Verfahren nach Anspruch 9; und
• Vergleichen der bestimmten Menge mit einem Referenzwert,
wobei, wenn die bestimmte Menge an Anti-PLA2R-Antikörpern in der Probe gleich dem oder größer als der Referenzwert ist, dies angibt, dass das Subjekt von einem Schema zur Prävention oder Behandlung von Nierenerkrankung unter Verwendung eines Bindungspartners für einen Anti-PLA2R-Antikörper profitiert.

13. Verfahren nach Anspruch 12, wobei:
i) das Subjekt eine Einzelperson ist, bei der Nierenerkrankung diagnostiziert wurde, wobei die Ursache der Nierenerkrankung nicht bestimmt wurde; und/oder
ii) das Verfahren ferner das Erhalten eines Wertes umfasst, der für die Menge an Anti-PLA2R-Antikörpern in der Körperflüssigkeit des Subjekts repräsentativ ist, und das Vergleichen dieses erhaltenen Wertes mit einem Referenzwert, wobei, wenn der erhaltene Wert größer als der Referenzwert ist, dies angibt, dass das Subjekt von einem Schema zur Prävention oder Behandlung von Nierenerkrankung unter Verwendung eines Peptids nach einem der Ansprüche 1 bis 3 profitiert.

14. Verfahren nach Anspruch 9 zur Verwendung beim Überwachen der Wirksamkeit bei einem Subjekt eines Behandlungsschemas zur Prävention oder Behandlung von Nierenerkrankung, wobei das Verfahren Folgendes umfasst:
• Untersuchen einer Probe einer Körperflüssigkeit eines Subjekts, das sich einem Behandlungsschema zur Prävention oder Behandlung von Nierenerkrankung unterzieht, um die Menge an Anti-PLA2R-Antikörpern, die in der Körperflüssigkeitsprobe des Subjekts vorhanden ist, durch ein Verfahren nach Anspruch 9 zu bestimmen;
• Vergleichen der bestimmten Menge mit einem Referenzwert; und
• basierend auf diesem Vergleich, Bestimmen, ob das Behandlungsschema des Subjekts zur Prävention oder Behandlung von Nierenerkrankung wirksam ist.

15. Verfahren nach Anspruch 14, wobei:
i) der Referenzwert der Mittelwert plus drei Standardabweichungen der Menge an Anti-PLA2R-Antikörpern in ungefähr 30 Proben von gesunden gesunden Einzelpersonen ohne Nierenerkrankung ist oder der Referenzwert repräsentativ für die Menge an Anti-PLA2R-Antikörpern ist, die in einer vergleichbaren Probe von dem Subjekt zu einem früheren Zeitpunkt vorhanden sind;
ii) wobei das Subjekt ein Patient ist, der sich Therapie für Nierenerkrankung unterzieht; oder
iii) wobei das Subjekt ein Teilnehmer an einer klinischen Studie ist.

## Revendications

1. Peptide constitué d'une longueur allant jusqu'à 40 résidus d'acides aminés, le peptide comprenant une séquence d'acides aminés K-X1-X2-X3-X4-X5-K-X6-X7-X8-X9-X10-X11-X12-X13-K (SEQ ID n° : 2), dans laquelle X1 et X13 représentent tous les deux des résidus cystéine et dans laquelle la séquence d'acides aminés décrite dans SEQ n° : 2 partage une identité d'au moins 80 % avec SEQ ID n° : 3 sur toute la longueur de la séquence.

2. Peptide selon l'une quelconque des revendications précédentes présentant une longueur de 31 résidus d'acides aminés.

3. Peptide selon la revendication 1 ou la revendication 2 : ledit peptide :
i) comprenant des résidus d'acides aminés supplémentaires situés au niveau de la terminaison N de SEQ ID n° : 2 ;
ii) comprenant un fragment de PLA2R comprenant la séquence à partir des résidus d'acides aminés 50 à 65 de la protéine pleine longueur (SEQ ID n° : 3) ;
iii) étant constitué de SEQ ID n° : 3 ;
iv) comprenant ou étant constitué d'un fragment de PLA2R comprenant la séquence à partir des résidus d'acides aminés 35 à 65 de la protéine plaine longueur (SEQ ID n° : 4) ;
v) comprenant au moins une séquence d'acides aminés supplémentaire dans le groupe constitué par : une séquence provenant du domaine fibronectine II de PLA2R ; une séquence provenant d'un domaine CTLD de PLA2R, telle qu'une séquence provenant du domaine CTLD1 de PLA2R, une séquence provenant du domaine CTLD2 de PLA2R, et une séquence provenant du domaine CTLD3 de PLA2R,
éventuellement :
i) ladite séquence provenant du domaine fibronectine II de PLA2R comprenant la séquence EDDLLWCATTSR ; ou
ii) ladite séquence provenant du domaine CTLD de PLA2R comprenant une séquence choisie dans le groupe constitué par : YLNHIDHEIVEKDAWK ; YYATHCEPGWNPYNR ; TWHEALR ; AGHVLSDAESGCQEGWER ; et YSGGCVAMRGR ;
vi) partageant une identité d'au moins 80 % avec une SEQ ID n° : 4.

4. Peptide selon l'une quelconque des revendications 1 à 3 pour une utilisation dans la prévention ou le traitement d'une maladie rénale.

5. Composition pharmaceutique comprenant un peptide selon l'une quelconque des revendications 1 à 3 et un vecteur pharmaceutiquement acceptable.

6. Dispositif permettant le traitement extracorporel du sang d'un patient, le dispositif comprenant :
• un peptide selon l'une quelconque des revendications 1 à 3 ; et
• un moyen pour séparer le peptide du sang.

7. Dispositif selon la revendication 6 :
i) ledit moyen permettant de séparer la partenaire de liaison du sang comprenant un substrat d'un matériau choisi dans le groupe constitué par : la cellulose ; les dérivés de cellulose ; l'agarose ; les dérivés d'agarose ; la polysulfone ; les dérivés de polysulfone ; le polyacrylamide ; les dérivés de polyacrylamide ; et le nylon ; et/ou
ii) ledit dispositif étant fourni sous une forme choisie dans le groupe constitué par : les cassettes à fibres creuses, les membranes et les billes.

8. Peptide pour une utilisation selon la revendication 4 pour une utilisation dans le traitement ou la prévention d'une maladie rénale, ladite maladie rénale étant choisie dans le groupe constitué par : une insuffisance rénale primaire ; une néphropathie membraneuse, telle qu'une néphropathie membraneuse idiopathique ou une néphropathie membraneuse de novo ; et une glomérulosclérose segmentaire focale.

9. Procédé de détermination des taux d'anticorps anti-PLA2R chez un sujet, le procédé comprenant :
• la mise en contact d'un peptide selon l'une quelconque des revendications 1 à 3 avec un échantillon d'un fluide corporel provenant du sujet, de sorte que les anticorps anti-PLA2R présents dans l'échantillon de fluide corporel du sujet soient capables de se lier au peptide et d'être retenu par celui-ci ; et
• la détermination de la quantité d'anticorps anti-PLA2R retenue par le peptide.

10. Procédé selon la revendication 9 pour une utilisation dans le diagnostic d'une maladie rénale chez un sujet, le procédé comprenant :
• la détermination *in vitro* de la quantité d'anticorps anti-PLA2R dans un échantillon de fluide corporel provenant d'un sujet dans un procédé selon la revendication 9 ; et
• la comparaison de la quantité déterminée à une valeur de référence,
si la quantité déterminée d'anticorps anti-PLA2R dans l'échantillon est supérieure ou égale à la valeur de référence, ceci indique que le sujet souffre d'une maladie rénale.

11. Procédé selon la revendication 10, ledit sujet étant un sujet qui est considéré comme présentant un risque de maladie rénale.

12. Procédé selon la revendication 9 pour une utilisation dans la sélection d'un régime approprié pour la prévention ou le traitement d'une maladie rénale, le procédé comprenant :
• la détermination de la quantité d'anticorps anti-PLA2R dans un échantillon de fluide corporel provenant d'un sujet dans un procédé selon la revendication 9 ; et
• la comparaison de la quantité déterminée à une valeur de référence,
si la quantité déterminée d'anticorps anti-PLA2R dans l'échantillon est supérieure ou égale à la valeur de référence, ceci indique que le sujet bénéficiera d'un régime pour la prévention ou le traitement d'une maladie rénale utilisant un partenaire de liaison pour un anticorps anti-PLA2R.

13. Procédé selon la revendication 12 :
i) ledit sujet étant un individu diagnostiqué comme souffrant d'une maladie rénale, où la cause de la maladie rénale n'a pas été déterminée ; et/ou
ii) ledit procédé comprenant en outre l'obtention d'une valeur représentative de la quantité d'anticorps anti-PLA2R dans le fluide corporel du sujet, et la comparaison de cette valeur obtenue à une valeur de référence, si la valeur obtenue est plus grande que la valeur de référence ceci indiquant que le sujet bénéficiera d'un régime pour la prévention ou le traitement d'une maladie rénale qui utilise un peptide selon l'une quelconque des revendications 1 à 3.

14. Procédé selon la revendication 9 pour une utilisation dans la surveillance de l'efficacité chez un sujet d'un régime de traitement pour la prévention ou le traitement d'une maladie rénale, le procédé comprenant :
• l'analyse d'un échantillon d'un fluide corporel d'un sujet subissant un régime de traitement pour la prévention ou le traitement d'une maladie rénale, pour déterminer la quantité d'anticorps anti-PLA2R présente dans l'échantillon de fluide corporel du sujet par un procédé selon la revendication 9 ;
• la comparaison de la quantité déterminée à une valeur de référence ; et
• sur la base de cette comparaison, la détermination pour savoir si le régime de traitement du sujet est efficace pour la prévention ou le traitement d'une maladie rénale.

15. Procédé selon la revendication 14 :
i) ladite valeur de référence étant la moyenne plus trois écarts types de la quantité d'anticorps anti-PLA2R dans approximativement 30 échantillons provenant d'individus sains normaux sans maladie rénale ou ladite valeur de référence étant représentative de la quantité d'anticorps anti-PLA2R présente dans un échantillon comparable provenant du sujet à un moment antérieur ;
ii) ledit sujet étant un patient subissant une thérapie pour une maladie rénale ; ou
iii) ledit sujet étant un participant à une essai clinique.
